(19) Europäisches Patentamt
European Patent Office
Office européen des brevets

(11) Veröffentlichungsnummer : **0 086 457 B1**

(12) **EUROPÄISCHE PATENTSCHRIFT**

(45) Veröffentlichungstag der Patentschrift : **30.11.88**

(21) Anmeldenummer : **83101246.3**

(22) Anmeldetag : **10.02.83**

(51) Int. Cl.[4] : **C 07 F 17/00**, C 07 F 15/00, C 07 F 11/00, C 07 F 7/28, C 07 F 9/02

(54) **Herstellung von Übergangsmetallkomplexen.**

(30) Priorität : **17.02.82 DE 3205550**

(43) Veröffentlichungstag der Anmeldung : **24.08.83 Patentblatt 83/34**

(45) Bekanntmachung des Hinweises auf die Patenterteilung : **30.11.88 Patentblatt 88/48**

(84) Benannte Vertragsstaaten : **AT BE CH DE FR GB IT LI LU NL SE**

(56) Entgegenhaltungen :
**EP-A- 0 003 564**
**EP-A- 0 009 685**
**DE-B- 1 059 452**
**US-A- 3 088 961**
**Anjew. Chemie Int. Ed. 24 (1985) 248-262**

(73) Patentinhaber : **Studiengesellschaft Kohle mbH**
**Kaiser-Wilhelm-Platz 1**
**D-4330 Mülheim/Ruhr (DE)**

(72) Erfinder : **Bönnemann, Helmut, Dr.**
**Grashofstrasse 82**
**D-4330 Essen/Ruhr (DE)**
Erfinder : **Bogdanovic, Borislav, Dr.**
**Kaiser-Wilhelm-Platz 1**
**Mülheim/Ruhr (DE)**

(74) Vertreter : **von Kreisler, Alek, Dipl.-Chem. et al**
**Deichmannhaus am Hauptbahnhof**
**D-5000 Köln 1 (DE)**

**EP 0 086 457 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Vorliegende Erfindung betrifft ein allgemein anwendbares Verfahren zur Synthese von Übergangsmetall-Komplexen durch Umsetzung von Übergangsmetallverbindungen in Gegenwart von komplexbildenden Elektronendonatoren mit speziell aktiviertem Magnesium. Es sind mehrere Verfahren zur Herstellung von Übergangsmetall-Komplexen unter Anwendung von reduzierenden Alkali- und Erdalkalimetallen beschrieben worden, wobei Magnesium unter den Gesichtspunkten von Wirtschaftlichkeit, untoxischer und ungefährlicher Handhabung sowie Verfügbarkeit besonders vorteilhaft ist.

So findet sich die Umsetzung von Nickelsalzen mit Magnesiumgrieß in Gegenwart von Triphenylphosphan bereits in der DE-B-11 26 864. Der dabei entstehende Nickel-Komplex kann jedoch nicht isoliert werden, sondern wird in situ als Katalysator eingesetzt. Die DE-A-14 43 461 beschreibt die Herstellung eines Nickel-Katalysators durch Umsetzung von Nickelverbindungen in Gegenwart von 1,3-Dienen u. a. mit Magnesium-Metall. Auch in diesem Fall wird kein Nickel-Olefinkomplex gefaßt. Eine in-situ-Umsetzung von Nickelverbindungen mit Magnesium in Gegenwart von Phosphanen beschreibt auch DE-A-22 21 113, wobei jedoch keine Komplexverbindung des Nickels in Substanz charakterisiert wird. Zur Herstellung von Übergangsmetall-Komplexverbindungen definierter Zusammensetzung sind diese Verfahren offenbar nicht brauchbar. Das italienische Patent 887 228 (C.A. 83, 28373) enthält die Herstellung von Bis-(Cyclo-(1,5)-octadien) Nickel aus Nickelhalogeniden und Cyclo-(1,5)-octadien durch Umsetzung u. a. mit Magnesium. Das Produkt ist jedoch zersetzlich und bedarf stabilisierender Zusätze.

Für technische Anwendungen sind jedoch lagerfähige Reinsubstanzen erwünscht. Klein und Karsch berichten über die Synthese von Tetrakis(trimethylphosphan) Cobalt (0) mit Magnesiummetall als Reduktionsmittel bei 24 h Reaktionszeit. (H.-F. Klein u. H. H. Karsch, Chem. Ber. 108, 944-955 (1975)). Schließlich verwenden G. Wilke und W. Gausing Magnesiummetall für die reduktive Synthese von Tris(butadien) molybdän und -wolfram (Angew. Chem. 93, 201-202 (1981)). Die Reaktionszeit beträgt 48 h. Für technische Anwendungen sind jedoch schnellere Herstellverfahren erforderlich. Außerdem hängt die Reaktivität von Magnesiummetall stark von der Größe und Reinheit seiner aktiven Oberfläche ab (vgl. z. B. J. R. Blackborow, D. Young : « Metal Vapour Synthesis in Organometallic Chemistry », Springer-Verlag 1979 s. 179). Bis heute steht kein befriedigendes Verfahren zur Verfügung, das die wirtschaftliche Herstellung von Übergangsmetallkomplexen auf der Basis von Magnesium als Reduktionsmittel gestattet.

Es hat nicht an Versuchen gefehlt, die Reaktivität von Magnesiummetall durch Zusatz von Aktivatoren und Beschleunigern zu verbessern (vgl. z. B. Y.-H. Lai, Synthesis 1981, 586). So wurde beispielsweise in der EP-A-0 003 564 ein Verfahren zur Herstellung von Magnesiumhydriden durch Umsetzung von metallischem Magnesium mit molekularem Wasserstoff in Gegenwart eines Katalysators vorgeschlagen, der eine Verbindung eines Metalls der vierten bis achten Nebengruppe des Periodensystems sowie eine metallorganische Verbindung bzw. ein Hydrid eines Elements der ersten bis dritten Hauptgruppe umfaßt. Dem System wurde außerdem ein Aktivator in Form eines polycyclischen Aromaten oder eines tertiären Amins zugesetzt.

Auch für die heterogene Reaktion zwischen Übergangsmetallsalzen und Magnesium wurden solche Beschleuniger bzw. Aktivierungsmethoden gelegentlich angewandt. So beschreiben die DE-A-23 53 198 und 23 53 240 ein Verfahren zur Herstellung eines nullwertigen Nickel-Komplexes unter Einsatz u. a. von Magnesium und Zinkhalogeniden bzw. Ammonhalogeniden als beschleunigern in organischen Nitrilen als Lösungsmittel. Für technische Zwecke sind Nitrile jedoch als Lösungsmittel allgemein nicht wirtschaftlich, und der Zusatz von Fremdmetallsalzen wie Zinkhalogeniden erschwert die Reinigung der Produkte.

Zur Reduktion von bestimmten Cyclopentadienyl-Übergangsmetallhalogeniden mit Magnesium verwenden M. D. Rausch und D. J. Sikora (J. Am. Chem. Soc. 103, 1265-1267 (1981)) Quecksilberchlorid als Aktivator oder setzen ein Magnesium ein, das mit Kaliummetall aus Magnesiumchlorid (vgl. R. E. Rieke et al., J. Am. Chem. Soc. 96, 1775-1781 (1974) erzeugt wird. Diese Aktivierungsverfahren sind technisch extrem aufwendig oder erfordern den Einsatz von hochgiftigem Quecksilbersalz.

Überraschend wurde nun gefunden, daß der Zusatz einer katalytischen Menge von Anthracen oder seinen eine oder zwei Methylgruppen oder Phenylgruppen enthaltender Derivate und/oder Magnesiumanthracen zu Magnesium ein hochaktives und zugleich ebenso kostengünstiges wie leicht handhabbares Reduktionsmittel erzeugt, das allgemein die wirtschaftliche Umsetzung von Übergangsmetallverbindungen in Gegenwart von komplexbildenden Liganden zu Komplexverbindungen der Übergangsmetalle gestattet.

Zur Herstellung der Komplexverbindungen nach dem erfindungsgemäßen Verfahren wird zunächst Magnesium-Metallpulver — vorzugsweise der Korngröße $< 0,15$ mm — in einem Lösungsmittel — bevorzugt Tetrahydrofuran oder Diglyme — mit 1-10 Mol%, vorzugsweise 2-6Mol%, Anthracen versetzt. Die Zugabe eines Alkylhalogenids wie Methyljodid ist dabei zweckmäßig, für das erfindungsgemäße Verfahren aber nicht notwendig. Nach Ablauf von 1-3 Stunden erhält man neben einer kleinen Menge Magnesiumanthracen ein hochaktives Magnesium, das zur Reduktion der Übergangsmetallsalze in Gegenwart komplexbildender Liganden bei milden Bedingungen eingesetzt werden kann. Nach einer Variante des erfindungsgemäßen Verfahrens setzt man vorgefertigtes Magnesiumanthracen als Aktivator zu. Die Ausführung der Aktivierung im Ultraschallbad begünstigt die Erzeugung einer blanken Metalloberfläche. Erfindungsgemäß bringt man die Übergangsmetallsalze sowie die komplexbildenden Liganden mit dem Magnesium/Anthracen-System bzw. Magnesiumanthracen bei Temperaturen von — 78 °C bis + 150 °C, vorzugsweise — 30 °C bis + 80 °C, in Berührung, wobei die Reaktion unter Wärmeentwicklung abläuft und binnen Minuten bis maximal 3 Stunden beendet ist.

Dabei ist von Bedeutung, daß Anthracen bzw. Magnesiumanthracen während der Umsetzung des Magnesiums mit den betreffenden Übergangsmetallsalzen als Katalysatoren für die Erzeugung hochreaktiver Mg-Spezies wirken. Die Weglassung dieser Aktivierungs-Katalysatoren führt zu erheblich schlechteren Komplexausbeuten oder zur unvollständigen Reduktion des Ausgangsproduktes wie Vergleichsversuche (z. B. Beispiele 7, 8 und 29) zeigen. Wie für einen Katalysator typisch, kann das leicht abtrennbare Anthracen aus den Ansätzen wiedergewonnen und erneut verwendet werden (Beispiel 22).

Es entspricht einer weiteren Ausführungsform des erfindungsgemäßen Verfahrens, Übergangsmetallsalze in Gegenwart komplexbildender Liganden zu Organoübergangsmetallkomplexen mit Magnesium umzusetzen, dem eine katalytische Menge eines Anthracenderivats zugesetzt wurde, das eine oder zwei Methylgruppen oder Phenylgruppen als Substituenten enthält. In gleicher Weise ist es möglich, anstelle des Anthracenderivats allein oder auch zusammen mit diesem ein Addukt an Magnesium als Aktivator für das metallische Magnesium zuzusetzen. So können beispielsweise Cobaltsalze, wie Cobalt(III) acetylacetonat, in Gegenwart von Inden und Cycloocta-1.5-dien in hoher Ausbeute zu $\eta^5$-Indenylcobalt-cycloocta-1.5-dien-Komplexen umgesetzt werden. Auch in diesen Fällen kann das in katalytischen Mengen eingesetzte Anthracenderivat aus den Reaktionsmischungen wiedergewonnen und erneut verwendet werden.

Als Übergangsmetalle werden bevorzugt die Elemente der Gruppen IVB bis VIIIB des Periodensystems der Elemente eingesetzt, als Übergangsmetallsalze bzw. -verbindungen solche, die entweder anorganische oder organische Anionen enthalten, vorzugsweise jene, die in den als Lösungsmittel verwendeten Systemen solvatisiert werden, wie Halogenide, Alkoholate und Salze organischer Säuren. Beispiele von Übergangsmetallen der Gruppen IVB, VB, VIB, VIIB und VIIIB des Periodensystems sind Titan, Zirkon, Hafnium ; Vanadin, Niob, Tantal ; Chrom, Molybdän, Wolfram ; Mangan, Techneticum, Rhenium ; Eisen, Cobalt, Nickel, Ruthenium, Rhodium, Palladium, Osmium, Iridium, Platin.

Als Komplexbildner können allgemein elektronenabgebende Stoffe eingesetzt werden, die mit Übergangsmetallen Durchdringungskomplexe bilden. Als solche kommen Verbindungen mit C-C-Mehrfachbindungen in Frage wie Olefine, z. B. Ethylen, aber auch substituierte Olefine, wie Methyl cyclopropan, Stilben, Maleinsäureanhydrid oder Acrylnitril, cyclische Olefine, wie Cyclopropenderivate, Norbornen, cyclische Mehrfacholefine, wie Cyclooctadien, Cyclopentadien und -Derivate, Cyclododecatrien, aber auch Mehrfacholefine, wie Allen und konjugierte Olefinsysteme, wie 1,3-Diene (z. B. Butadien, Isopren, Methylheptatrien), Sorbinsäureester, ferner Alkine, wie Acetylen, Butin-2, Tolan, und Cycloalkine, wie z. B. Cyclododecin. Außerdem eignen sich Liganden mit freien Elektronenpaaren wie Phosphane, Phosphite, Arsane, Triorganylantimonverbindungen, ferner Pyridine und Dipyridine sowie Kohlenmonoxid, außerdem Kombinationen verschiedener Komplexbildner.

Das erfindungsgemäße Verfahren ermöglicht es erstmals, das kostengünstige und gefahrlos handhabbare sowie untoxische Magnesium zur Herstellung von Übergangsmetallkomplexen allgemein und mit gutem Wirkungsgrad einzusetzen. Dabei lassen sich auf der Basis von Magnesium als Reduktionsmittel folgende Klassen von Komplex-Verbindungen der Übergangsmetalle mit besonderem Vorteil erhalten : $\eta^5$-Cyclopentadienyl-Übergangsmetallkomplexe (Metallocene) ; $\eta^5$-Cyclopentadienyl-Übergangsmetallkomplexe (Metallocene) ; $\eta^5$-Cyclopentadienyl-Cobalt-Olefin-Halbsandwich-Verbindungen, die nach den bisher bekannten Methoden in einer Stufe nicht zu erhalten sind ; $\eta^3$-Allyl-Komplexe der Übergangsmetalle, vorzugsweise des Nickels und Cobalts, in Gegenwart von (1,3)-Dienen ; Olefinkomplexe, insbesondere Bis(Cyclooctadien)-Komplexe und Butadienkomplexe nullwertiger Übergangsmetalle wie Ni, Pd, Pt und Mo ; $MTL_n$-Komplexe der Übergangsmetalle, wobei MT für ein nullwertiges Übergangsmetall steht, L einen Neutralliganden wie Phosphan oder Phosphit bedeutet und n ein Zahlenindex zwischen 2 und 5 ist ; Metallocendihalogenide, insbesondere $\eta^5$-Cyclopentadienyltitandihalogenide. Die erfindungsgemäß kostengünstig herzustellen Komplexverbindungen werden z. B. als Katalysatoren in der technischen Chemie verwendet (P. W. Jolly in : Ullmann's Enzyklopädie der Technischen Chemie, Band 16, S. 587 f.) Ferrocen wird als Zusatz zur raucharmen Verbrennung von Mineralölen verwendet.

Cobaltocene sowie Cyclopentadienyl-Cobalt-Halbsandwich-Komplexe dienen als Katalysatoren zur Herstellung von Pyridinderivaten aus Alkinen und Nitrilen [s. H. Bönnemann, Angew. Chemie 90, 517 (1978), sowie US-PS 4 006 149 ; DE-C 28 40 460], ferner die in vorliegender Anmeldung gegebenen Anwendungsbeispiele 16, 17, 18, 19, 20 und 21 zur Herstellung von Pyridinderivaten.

Biscycloocta-(1,5) diennickel findet breite Anwendung in der Synthese von Nickelkomplexen durch Ligandenaustausch [Inorg. Synth. XV, 5 (1974) sowie als Katalysator in der organischen Synthese, zumal bei der Herstellung von Naturstoffen [P. W. Jolly und G. Wilke : The Organic Chemistry of Nickel, Vol. II, Academic Press (1975), ferner P. W. Jolly, G. Wilke in Merck Kontakte, 21, (1974)] Tetrakistriphenylphosphanpalladium findet als Katalysator bei der Funktionalisierung von Olefinen Verwendung [J. Tsuji in : Adv. in organomet. Chem. 17 (1979) S. 141 f] Phosphitkomplexe des nullwertigen Nickels ermöglichen die katalytische Hydrocyanierung von Butadien zu Adipinsäurenitril im industriellen Maßstab [Chem. Eng. News, 26. Apr. 1971, 30 ; Chem. Week, 12. Mai 1971, 32-37] Metallocendihalogenide z. B. des Titans finden

darüber hinaus Interesse als potentielle Cytostatika (vergl. H. Köpf und P. Köpf-Maier, Nachr. Chem. Techn. Lab. 29 (1981) S. 154).

Beispiel 1

Cobaltocen

14,4 g (600 mMol) Magnesiumpulver, Korngröße < 0,15 mm, werden unter Schutzgas mit 1,1 g (6,2 mMol) Anthracen, 300 ml THF und 0,1 ml Methyljodid versetzt. Es bildet sich unter Rühren bei 20 °C eine gelbgrüne Lösung, aus der sich nach ca. 2 Stunden orange gefärbtes Magnesium-Anthracen ausscheidet. Man behandelt die Reaktionsmischung ca. 3 Stunden im Ultraschallbad (HF-Dauerspitzenleistung 240 Watt, 35 kHz) und erwärmt anschließend unter Rühren auf 60 °C. Nach Zugabe von 19,8 g (300 mMol) monomerem Cyclopentadien entfernt man die Wärmequelle und dosiert innerhalb 20 Minuten 35,6 g (100 mMol) festes Cobalt(III) acetylacetonat zu. Dabei verfärbt sich die Reaktionsmischung tiefbraun unter heftiger Wärmetönung und kommt zum Rückfluß (66 °C). Nach Abkühlen auf 20 °C filtriert man von nicht reagiertem Magnesium über eine G-3-Glasfritte ab und engt das klare, tiefbraune Filtrat im Hochvakuum (1,3.$10^{-3}$ mbar ; $10^{-3}$ Torr) bis zur Trockne ein (Badtemp. : max. 30 °C). Den Rückstand nimmt man in 500 ml Pentan auf und filtriert vom Unlöslichen über eine G-3-Glasfritte ab. Den Filterkuchen wäscht man mehrere mit insgesamt 500 ml Pentan, bis das Filtrat nahezu farblos ist. Das klare, rotbraune Filtrat engt man auf ca. 200 ml ein und läßt den Komplex bei —80 °C auskristallisieren. Die überstehende Mutterlauge drückt man ab, wäscht 2-3 mal mit ca. 50 ml —80 °C kaltem Pentan. Nach Trocknen im Vakuum (0,13 mbar ; 0,1 Torr) erhält man 12,4 g (63,7 mMol = 63,7 % d. Th.) reines Cobaltocen in tief schwarzvioletten Kristallen viom Smp. 172,5 °C, unter Schutzgas gemessen.

Beispiel 2

Bis-(Indenyl)-Cobalt

14,4 g (600 mMol) Magnesiumpulver, Korngröße < 0,15 mm, werden unter Schutzgas mit 1,1 g (6,2 mMol) Anthracen, 350 ml THF und 0,1 ml Methyljodid versetzt. Es bildet sich unter Rühren bei 23 °C eine gelbgrüne Lösung, aus der sich nach 100 Minuten orange gefärbtes Magnesium-Anthracen ausscheidet. Man behandelt die Reaktionsmischung ca. 3 Stunden im Ultraschallbad (HF-Dauerspitzenleistung 240 Watt, 35 kHz) und erwärmt anschließend unter Rühren auf 60 °C. Nach Zugabe von 34,8 g (300 mMol) Inden entfernt man die Heizung und dosiert innerhalb 40 Minuten 35,6 g (100 mMol) festes Cobalt(III) acetylacetonat zu. Dabei färbt sich die Reaktionsmischung unter Anstieg der Temperatur auf 68 °C tiefbraun. Nach Abkühlen auf 23 °C filtriert man vom überschüssigen Magnesium über eine G-3-Glasfritte ab und engt das klare braunrote Filtrat im Hochvakuum (1,3 · $10^{-3}$ mbar ; $10^{-3}$ Torr) bis zur Trockne ein (Badtemp. : max. 30 °C). Den Rückstand nimmt man in 500 ml Pentan auf und filtriert vom Unlöslichen über eine G-3-Glasfritte ab. (Den Filterkuchen wäscht man mehrere Male mit insgesamt 500 ml Pentan, bis das Filtrat nahezu farblos ist). Das klare, rotbraune Filtrat engt man auf ca. 200 ml ein und läßt den Komplex bei —80 °C auskristallisieren. Die überstehende Mutterlauge drückt man ab, wäscht 2-3 mal mit ca. 50 ml —80 °C kaltem Pentan. Nach Trocknen im Vakuum (0,13 mbar ; 0,1 Torr) erhält man 10,3 g (35,5 mMol = 35,5 % d. Th.) Bis-(Indenyl) Cobalt in glitzernden schwarzen Kristallen.

Beispiel 3

$\eta^5$-Indenyl-$\eta^5$ cyclopentadienyl-Cobalt

7,2 g (300 mMol) Magnesiumpulver, Korngröße < 0,15 mm, versetzt man unter Schutzgas mit 1,1 g (6,2 mMol) Anthracen, 300 ml THF und 0,1 ml Methyljodid. Es bildet sich unter Rühren bei 20 °C eine gelbgrüne Lösung, aus der sich nach ca. 2 Stunden orange gefärbtes Magnesium-Anthracen ausscheidet. Man behandelt die Reaktionsmischung etwa 3 Stunden lang im Ultraschallbad (Gerät siehe Beispiel 1) und erwärmt anschließend unter Rühren auf 60 °C. Nach Zugabe von 34,3 g (295,7 mMol) Inden und 7,7 g (116,7 mMol) monomerem Cyclopentadien entfernt man die Wärmequelle und dosiert innerhalb von 20 Minuten 35,6 g (100 mMol) festes Cobalt-III-acetylacetonat zu. Dabei verfärbt sich die Reaktionsmischung unter heftiger Wärmetönung (Temperatur bis 70 °C) tiefrotbraun. Nach Abkühlen auf 23 °C filtriert man von evtl. unlöslichem und nicht reagiertem Magnesium über eine G-3-Glasfritte ab und engt das klare rotbraune Filtrat im Vakuum (1,3 · $10^{-2}$ mbar ; $10^{-2}$ Torr) ein, bis die Sublimation einer roten Verbindung beobachtet wird. Man hebt das Vakuum auf, nimmt den hochviskosen, nahezu schwarzen Rückstand in ca. 400 ml Pentan auf und filtriert vom Unlöslichen über eine G-3-Fritte ab. Das Filtrat engt man im Vakuum (0,13 mbar ; $10^{-1}$ Torr) bis zur Trockne ein und sublimiert aus dem violettbraunen Rückstand im Hochvakuum (1,3 · $10^{-3}$ mbar ; $10^{-3}$ Torr) bei einer Badtemperatur von 30-150 °C eine scharlachrote Verbindung heraus. Das Sublimat wird in ca. 100 ml Pentan gelöst, evtl. vom Unlöslichen über eine G-3-Fritte befreit und das klare, tiefrote Filtrat auf —80 °C abgekühlt. Vom angefallenen Komplex drückt man die überstehende Mutterlauge ab und wäscht die Kristalle 2 mal mit je 25 ml

— 80 °C kaltem Pentan. Nach Trocknen im Vakuum (0,13 mbar ; $10^{-1}$ Torr) erhält man 4,2 g $\eta^5$-Indenyl-$\eta^5$ cyclopentadienyl-Cobalt (17,6 mMol = 17,6 % d. Th.), scharlachrote Nadeln vom Smp. 173 °C.

Massenspektrum :
m/e : 239 ($M^-$) ; 174, 124 ; 59.

Beispiel 4

Ferrocen

Man versetzt gemäß Beispiel 1 3,6 g Magnesiumpulver in 300 ml THF mit 0,6 g (3,4 mMol) Anthracen, erwärmt die orangegefärbte Reaktionsmischung auf 65 °C, versetzt mit 19,8 g (300 mMol) monomerem Cyclopentadien und dosiert innerhalb 1 Stunde 16,2 g (100 mMol) $FeCl_3$ zu. Unter heftiger Wärmetönung färbt sich dabei das Reaktionsgemisch gelbbraun. Nach Abkühlen auf 23 °C engt man die Reaktionsmischung im Vakuum bis zur Trockne ein und extrahiert den Rückstand mit insgesamt 500 ml Pentan. Den dunkelgelb gefärbten Extraktkühlt man auf — 80 °C ab und kristallisiert Ferrocen aus. Nach Trocknen im Vakuum erhält man 12,9 g = 69 % d. Th. Ferrocen.

Beispiel 5

$\eta^5$-Cyclopentadienyl-Cobalt-Cyclopentadien

7,2 g (300 mMol) Magnesiumpulver, Korngröße < 0,15 mm, werden unter Schutzgas unter Rühren bei 20 °C mit 1,1 g (6,2 mMol) Anthracen, 300 ml THF und 0,1 ml Methyljodid versetzt. Nach ca. 2 Stunden aktiviert man ca. 3 Stunden lang die Reaktionsmischung im Ultraschallbad (vgl. Beispiel 1). Anschließend bringt man die Mischung unter Rühren auf 60 °C. Nach Zugabe von 52,8 g (800 mMol) monomerem Cyclopentadien entfernt man die Wärmequelle und dosiert innerhalb 30 Minuten 35,6 g (100 mMol) festes Cobalt-III-acetylacetonat zu. Dabei verfärbt sich die Reaktionsmischung unter starker Wärmetönung (65 °C) tiefrotbraun. Nach Abkühlen auf 23 °C filtriert man vom evtl. Unlöslichen über eine G-3-Glasfritte ab und engt das tiefrotbraune Filtrat ein im Vakuum (0,13 mbar ; $10^{-1}$ Torr) bis zur Trockne (Badtemp. : max. 30 °C). Den Rückstand nimmt man in 500 ml Pentan auf und filtriert vom Unlöslichen ab. Das klare tiefrote Filtrat engt man im Vakuum (0,13 mbar ; $10^{-1}$ Torr) (Badtemp. : max. 30 °C) bis zur Trockne ein und sublimiert den Rückstand bei ($1{,}3 \cdot 10^{-3}$ mbar ; $10^{-3}$ Torr) (Badtemp. bis 60 °C ; Beginn der Sublimation bei Badtemp. von 30 °C). Das Sublimat löst man in ca. 100 ml Pentan und kristallisiert den Komplex bei — 80 °C aus. Man erhält 11,9 g (62,6 mMol = 62,6 % d. Th.) $\eta^5$-Cyclopentadienyl-Cobalt-Cyclopentadien als weinrote Kristalle vom Smp. 98-99 °C.

Massenspektrum :
m/e : 190 ($M^-$).

Beispiel 6

$\eta^5$-Methylcyclopentadienyl-Cobalt-Methylcyclopentadien

7,2 g (300 mMol) Magnesiumpulver, Korngröße < 0,15 mm, werden unter Schutzgas mit 1,1 g (6,2 mMol) Anthracen, 300 ml THF und 0,1 ml Methyljodid versetzt. Es bildet sich unter Rühren bei Raumtemperatur eine gelbgrüne Lösung, aus der sich nach ca. 2 Stunden orange gefärbtes Magnesium-Anthracen ausscheidet. Man behandelt ca. 3 Stunden die Reaktionsmischung im Ultraschallbad (Gerät wie in Beispiel 1 angegeben). Anschließend erwärmt man unter Rühren auf 60 °C. Nach Zugabe von 32,0 g (400 mMol) monomerem Methylcyclopentadien entfernt man die Wärmequelle und dosiert innerhalb 30 Minuten 35,6 g (100 mMol) festes Cobalt(III)-acetylacetonat zu. Dabei verfärbt sich die Reaktionsmischung unter Wärmetönung bis zum Rückfluß (65 °C) tiefrotbraun. Nach Abkühlen auf 23 °C filtriert man von evtl. Unlöslichem über eine G-3-Glasfritte ab und engt das tiefrotbraune Filtrat im Vakuum (0,13 mbar ; $10^{-1}$ Torr) bis zur Trockne ein (Badtemp. : max. 30 °C). Den Rückstand nimmt man in 500 ml Pentan auf und filtriert vom Unlöslichen ab. Das klare, tiefrote Filtrat engt man im Vakuum (0,13 mbar ; $10^{-1}$ Torr) (Badtemp. : max. 30 °C) bis zur Trockne ein und destilliert den Rückstand bei ($1{,}3 \cdot 10^{-3}$ mbar ; $10^{-3}$ Torr) (Badtemp. : 80-140 °C). Man erhält 13,6 g (62,4 mMol = 62,4 % d. Th.) $\eta^5$-Methylcyclopentadienyl-Cobalt-Methylcyclopentadien als dunkelrotes Öl, das bei etwa — 5 °C fest wird.

Beispiele 7-21

Die Verwendung von durch Anthracen aktiviertem Magnesium zur Herstellung von Cyclopentadienylcobalt-diolefin-Halbsandwich-Derivaten ist in den Beispielen 9-21 tabellarisch zusammengefaßt.

Verfahrensweise bei den Ansätzen ist analog Beispiel 1, wobei zunächst das Magnesium mit

Anthracen versetzt und mit Ultraschall behandelt wird. Anschließend gibt man die olefinischen Komplexpartner gemeinsam zu und dosiert die Cobaltsalze zu der Mischung. Die Aufarbeitung erfolgt durch Lösungsmittelwechsel von THF bzw. Diglyme zu Pentan.

Folgende Abkürzungen werden in der Tabelle verwendet. Beispiele 7 und 8 sind Vergleichsversuche ohne Anthracen.

ac = Acetat
acac = Acetylacetonat
OEt = Ethylat
Cp = $\eta^5$-Cyclopentadienyl
CpH = Cyclopentadien
Ind = $\eta^5$-Indenyl
Me = Methyl
t-But = tertiär-Butyl
COD = Cycloocta-(1,5) dien
NBD = Norbornadien

Die mit Stern versehenen Komplexe sind bisher unbekannte Produkte und werden im Anschluß an die Tabelle beschrieben.

(Siehe Tabellen Seite 7 ff.)

18-- Anwendung von Trimethylsilyl-Cyclopentadienyl-Cobalt-Cycloocta-(1,5)-dien als Katalysator zur Umsetzung von Acetonitril mit Acetylen

0,0458 g (0,1507 mMol) Trimethylsilyl-Cyclopentadienyl-Cobalt-Cycloocta-(1,5)-dien löst man in 114,2 g (2,785 Mol) Acetonitril und saugt diese Lösung bei Raumtemperatur in einen 500 ml Edelstahlautoklaven mit innen angebrachter Schlangenkühlung. Man sättigt das Acetonitril bei 18 bar mit Acetylen, wodurch ca. 57,5 g (2,212 Mol) Acetylen zugegeben werden. Innerhalb von 72 min wird auf 150 °C aufgeheizt, wobei der Druck auf 46 bar ansteigt. Erhöhung der Reaktionstemperatur auf 212 °C innerhalb von 160 min läßt den Druck auf den Maximalwert von 52 bar ansteigen. Nach insgesamt 312 min Reaktionszeit bringt man das Druckgefäß binnen 45 min durch Innenkühlung mit Wasser auf 20 °C.

146,2 g Rohprodukt werden aus dem Autoklaven ausgefüllt und die flüchtigen Bestandteile bei ($1{,}3 \cdot 10^{-3}$ mbar; $10^{-3}$ Torr) abkondensiert, wobei 0,4 g Rückstand hinterbleiben. Das Kondensat 145,8 g enthält laut gaschromatographischer Analyse 80,88 g (1,973 Mol) Acetonitril, 52,58 g (0,565 Mol) 2-Picolin und 8,61 g (0,110 Mol) Benzol, entsprechend einer 39,4 %igen 2-Picolin-Lösung in Acetonitril. Das molare Verhältnis 2-Picolin/Benzol beträgt dabei 5,1 : 1 und die Ausbeute an 2-Picolin bezogen auf umgesetztes Acetonitril : 69,5 %.

Umsatz : 29,2 % Acetonitril.

Katalysator-Ausnutzung : 3 749 Mol 2-Picolin/g-Atom Cobalt bzw. 5 913,6 kg 2-Picolin/kg Cobalt.

Anwendung von Trimethylsilyl-Cyclopentadienyl-Cobalt-Cycloocta-(1,5)-dien zur Umsetzung von 2-Cyanopyridin zu 2,2′-Bipyridyl

0,0360 g (0,1190 mMol) Trimethylsilyl-Cyclopentadienyl-Cobalt-Cycloocta-(1,5)-dien und 52,4 g (0,5038 Mol) 2-Cyanopyridin löst man in 100 ml (87,9 g) Toluol und saugt diese Lösung bei Raumtemperatur in einen 500 ml Edelstahlautoklaven mit innen angebrachter Schlangenkühlung. Man sättigt die Lösung bei 16 bar mit Acetylen, wodurch ca. 21 g (0,808 Mol) Acetylen zugegeben werden. Innerhalb von 45 min wird auf 150 °C aufgeheizt, wobei der Druck im Autoklaven auf maximal 42 bar ansteigt. Binnen 120 min erhöht man die Reaktionstemperatur auf 202 °C. Nach insgesamt 165 min Reaktionszeit bringt man das Druckgefäß binnen 40 min durch Innenkühlung mit Wasser auf 20 °C.

144,5 g Rohprodukt werden aus dem Autoklaven ausgefüllt und die flüchtigen Bestandteile bei ($1{,}3 \cdot 10^{-3}$ mbar; $10^{-3}$ Torr) abkondensiert, wobei 1,0 g Rückstand hinterbleiben. Das Kondensat, 143,3 g enthält laut gaschromatographischer Analyse 87,8 g Toluol, 41,0 g (0,394 Mol) 2-Cyanopyridin, 11,46 g (0,073 Mol) 2,2′-Bipyridyl und 2,44 g (0,031 Mol) Benzol, entsprechend einer 11,5 %igen 2,2′-Bipyridyl-Lösung in Toluol. Das molare Verhältnis 2,2′-Bipyridyl/Benzol beträgt dabei 2,4 : 1 und die Ausbeute an 2,2′-Bipyridyl bezogen auf umgesetztes 2-Cyanopyridin : 66,4 %.

Umsatz : 21,8 % 2,2′-Bipyridyl.

Katalysator-Ausnutzung : 613 Mol 2,2′-Bipyridyl/g-Atom Cobalt bzw. 1 632,2 kg 2,2′-Bipyridyl/kg Cobalt.

* Charakterisierung der neu dargestellten Verbindungen in Tabelle 1 :

Nr. 16 Methyl-$\eta^5$-Cyclopentadienyl-Cobalt-Cycloocta-(1,5)-dien

Gelbbraune Kristalle vom Schmelzpunkt — 15 °C aus Pentan durch Einengen und Kristallisieren bei — 80 °C.

**Tabelle 1**

| Nr. | Komplex | Co-Verbindung mMol, Dosiergut | Reduktionsmittel/ Aktivator mMol/mMol | Cyclopenta-dienyl-Verbindung mMol | Olefin mMol | Lsgm./ Temp. °C | Ausbeute % |
|---|---|---|---|---|---|---|---|
| 7 | Ind Co COD | $CoCl_2$ 100/30 Min. | Mg mit $C_1$ akt. - 104 | Inden 245 | COD 257 | THF/30 | 6,0 |
| 8 | Ind Co COD | $CoCl_2$ 100/30 Min. | Mg mit $J_2$ akt. 100 | Inden 250 | COD 250 | THF/65 | 19,2 |
| 9 | Ind Co COD | $CoCl_2$ 100/20 Min. | Mg/Anthracen 108/6,2 | Inden 250 | COD 250 | THF/65 | 73,8 |
| 10 | Ind Co COD | Co $ac_2$ 100/35 Min. | Mg/Anthracen 200/6,2 | Inden 250 | COD 250 | THF/65 | 80,5 |
| 11 | Ind Co COD | Co $acac_3$ 100/40 Min. | Mg/Anthracen 300/6,2 | Inden 250 | COD 250 | THF/65 | 85,1 |
| 12 | Ind Co COD | Co $acac_3$ 100/35 Min. | Mg/Anthracen 300/6,2 | Inden 110 | COD 110 | THF/65 | 51,0 |
| 13 | Ind Co COD | Co $acac_3$ 100/30 Min. | Mg/Anthracen 300/6,2 | Inden 250 | COD 250 | Diglyme/ 70 - 88 | 70,2 |

Tabelle 1 (Fortsetzung)

| | | | | | | | |
|---|---|---|---|---|---|---|---|
| 14 | Cp Co COD | Co acac$_3$ | Mg/Anthracen | CpH | COD | THF/66 | 79,1 |
| | | 100/20 Min. | 300/6,2 | 111 | 250 | | |
| 15 | Cp Co COD | Co(OEt)$_2$ | Mg/Anthracen | CpH | COD | THF/65 | 45,1 |
| | | 100/40 Min. | 400/7,5 | 250 | 250 | | |
| 16* | MeCpCoCOD | Co acac$_3$ | Mg/Anthracen | MeCpH | COD | THF/67 | 71,1 |
| | | 100/35 Min. | 300/6,2 | 110 | 250 | | |
| 17* | t-ButCpCOD | Co acac$_3$ | Mg/Anthracen | t-ButCpH | COD | THF/65 | 41,7 |
| | | 50/15 Min. | 150/3,1 | 57 | 125 | | |
| 18++ | Sime$_3$CpCoCOD | CO acac$_3$ | Mg/Anthracen | Me$_3$SiCpH | COD | THF/65 | 70,0 |
| | | 100/20 Min. | 300/6,2 | 110 | 250 | | |
| 19* | PhenylCpCoCOD | Co acac$_3$ | Mg/Anthracen | PhenylCpH | COD | THF/69 | ca.40 |
| | | 100/16 Min. | 300/6,2 | 90 | 250 | | |
| 20* | Sime$_3$IndCoCOD | Co acac$_3$ | Mg/Anthracen | Sime$_3$-Inden | COD | THF/69 | 50,6 |
| | | 100/20 Min. | 300/6,2 | 115 | 250 | | |
| 21* | Ind Co NBD | Co acac$_3$ | Mg/Anthracen | Inden | NBD | THF/69 | 30,5 |
| | | 100/40 Min. | 300/6,2 | 250 | 250 | | |

$^1$H-NMR ($CDCL_3$, 80 Mhz) :

$\delta H_1$ : 4,58 (m)
$\delta H_2$ : 4,27 (m)
$\delta H_3$ : 3,22 (m)
$\delta H_4$ : 2,34 (m)
$\delta H_5$ : 1,57 (m)
$\delta H_6$ : 1,55 (s)

Massenspektrum :
m/e : 246 ($M^-$), 78 %) ; 216 (90 %) ; 138 (100 %) ; 59 (53 %).

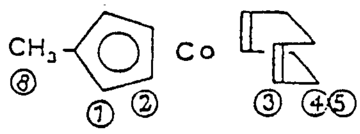

Anwendung als Katalysator

0,0364 g (0,1480 mMol) Methyl-Cyclopentadienyl-Cobalt-Cycloocta-(1,5)-dien löst man in 119,0 g (2,902 Mol) Acetonitril und saugt diese Lösung bei Raumtemperatur in einen 500 ml Edelstahlautoklaven mit innen angebrachter Schlangenkühlung. Man sättigt das Acetonitril bei 15 bar mit Acetylen, wodurch ca. 50,0 g (1,923 Mol) Acetylen zugegeben werden. Innerhalb von 37 min wird auf 140 °C aufgeheizt, wobei der Druck auf 48 bar ansteigt. Erhöhung der Reaktionstemperatur auf 180 °C innerhalb von 72 min beläßt den Druck auf dem Maximalwert von 48 bar. Nach insgesamt 120 min Reaktionszeit bringt man das Druckgefäß binnen 35 min durch Innenkühlung mit Wasser auf 22 °C.

129,7 g Rohprodukt werden aus dem Autoklaven ausgefüllt und die flüchtigen Bestandteile bei ($1,3 \cdot 10^{-3}$ mbar, $10^{-3}$ Torr) abkondensiert, wobei 0,1 g Rückstand hinterbleiben. Das Kondensat, 128,2 g, enthält laut gaschromatographischer Analyse 99,41 g (2,425 Mol) Acetonitril, 24, 16 g (0,260 Mol) 2-Picolin und 3,74 g (0,048 Mol) Benzol, entsprechend einer 19,6 %igen 2-Picolin-Lösung in Acetonitril. Das molare Verhältnis 2-Picolin/Benzol beträgt dabei 5,4 : 1 und die Ausbeute an 2-Picolin bezogen auf umgesetztes Acetonitril : 54,3 %.

Umsatz : 16,5 % Acetonitril.

Katalysatorausnutzung : 1757 Mol 2-Picolin/g-Atom Cobalt bzw. 2766,8 kg 2-Picolin/kg Cobalt.

Nr. 17$^-$ Tertiär-Butyl-$\eta^5$-Cyclopentadienyl-Cobalt-Cycloocta-(1,5)-dien
Dunkelbraune Kristalle vom Schmelzpunkt 45 °C.

Nach Vorreinigung durch Sublimation bei 80 °C bis 150 °C Badtemperatur und ($1,3 \cdot 10^{-3}$ mbar, $10^{-3}$ Torr) erhält man das Produkt aus Pentan bei — 80 °C. In Lösung merklich luftempfindlich.

Elementaranalyse :

| | | | |
|---|---|---|---|
| gefunden : | C 70,93 | H 8,62 | Co 20,52 % |
| berechnet : | C 70,82 | H 8,74 | Co 20,44 % |

$^1$H-NMR ($d_8$-Toluol, 80 Mhz) :

$\delta H_1$ : 4,60 (t ; J = 2,2 Hz)
$\delta H_2$ : 3,39 (t ; J = 2,2 Hz)
$\delta H_3$ : 3,41 (m)
$\delta H_4$ : 2,43 (m)
$\delta H_5$ : 1,65 (m)
$\delta H_6$ : 1,39 (s)

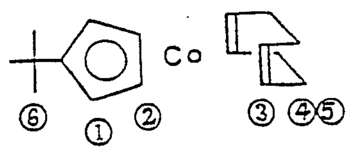

Massenspektrum :
m/e : 288 ($M^-$, 61 %) ; 231 (93 %) ; 229 (100 %) ; 164 (54 %) ; 137 (28 %) ; 125 (31 %) ; 59 (47 %).

Anwendung als Katalysator

0,0381 g (0,1321 mMol) t-Butyl-Cyclopentadienyl-Cobalt-Cycloocta-(1,5)-dien löst man in 116,25 g (2,835 Mol) Acetonitril und saugt diese Lösung bei Raumtemperatur in einen 500 ml Edelstahlautoklaven mit innen angebrachter Schlangenkühlung. Man sättigt das Acetonitril bei 15 bar mit Acetylen, wodurch ca. 54,0 g (2,077 Mol) Acetylen zugegeben werden. Innerhalb von 54 min wird auf 150 °C aufgeheizt, wobei der Druck auf 49 bar ansteigt. Erhöhung der Reaktionstemperatur auf 201 °C innerhalb von 120 min läßt den Druck auf den Maximalwert von 54,5 bar ansteigen. Nach insgesamt 120 min Reaktionszeit

bringt man das Druckgefäß binnen 45 min durch Innenkühlung mit Wasser auf 25 °C.

132,5 g Rohprodukt werden aus dem Autoklaven ausgefüllt und die flüchtigen Bestandteile bei ($1{,}3 \cdot 10^{-3}$ mbar ; $10^{-3}$ Torr) abkondensiert, wobei 0,4 g Rückstand hinterbleiben. Das Kondensat 132,0 g enthält laut gaschromatographischer Analyse 98,93 g (2,413 Mol) Acetonitril, 24,97 g (0,258 Mol) 2-Picolin und 4,55 g (0,0583 Mol) Benzol, entsprechend einer 20,2 %igen 2-Picolin-Lösung in Acetonitril. Das molare Verhältnis 2-Picolin/Benzol beträgt dabei 4,6 : 1 und die Ausbeute an 2-Picolin bezogen auf umgesetztes Acetonitril : 63,5 %.

Umsatz : 14,9 % Acetonitril

Katalysatorausnutzung : 2032 Mol 2-Picolin/g-Atom Cobalt bzw. 3203,8 kg 2-Picolin/kg Cobalt.

Nr. 19 Phenyl-$\eta^5$-Cyclopentadienyl-Cobalt-Cycloocta-(1,5)-dien. Nach Chromatographie auf einer 70 cm langen $SiO_2$-Säule (Eluens : Pentan) durch Auskristallisieren aus dem Eluens bei — 50 °C in Form kupferroter Blättchen vom Schmelzpunkt 64,3 °C.

Massenspektrum :
m/e : 308 ($M^-$, 98 %) ; 278 (68 %) ; 200 (100 %) ; 141 (64 %) ; 59 (42 %).

Anwendung als Katalysator

0,0403 g (0,1308 mMol) Phenyl-Cyclopentadienyl-Cobalt-Cycloocta-(1,5)-dien löst man in 116,3 g (2,115 Mol) Propionitril und saugt diese Lösung bei Raumtemperatur in einen 500 ml Edelstahlautoklaven mit innen angebrachter Schlangenkühlung. Man sättigt das Propionitril bei 14 bei mit Acetylen, wodurch ca. 40,5 g (1,558 Mol) Acetylen zugegeben werden. Innerhalb von 72 min wird auf 130 °C aufgeheizt, wobei der Druck auf 42 bar ansteigt. Trotz Erhöhung der Reaktionstemperatur auf 154 °C innerhalb von 59 min fällt der Druck auf 39 bar ab. Nach insgesamt 120 min Reaktionszeit bringt man das Druckgefäßt binnen 30 min durch Innenkühlung mit Wasser auf 20 °C.

128,5 g Rohprodukt werden aus dem Autoklaven ausgefüllt und die flüchtigen Bestandteile bei ($1{,}3 \cdot 10^{-3}$ mbar ; $10^{-3}$ Torr) abkondensiert, wobei 0,1 g Rückstand hinterbleiben. Das Kondensat 128,3 g enthält laut gaschromatographischer Analyse 103,9 g (0,226 Mol) Propionitril, 20,01 g (0,187 Mol) 2-Ethylpyridin und 2,60 g (0,033 Mol) Benzol, entsprechend einer 16,1 %igen 2-Ethylpyridin-Lösung in Propionitril. Das molare Verhältnis 2-Ethylpyridin/Benzol beträgt dabei 5,6 : 1 und die Ausbeute an 2-Ethylpyridin bezogen auf umgesetztes Propionitril : 82,7 %.

Umsatz : 10,7 % Propionitril

Katalysatorausnutzung : 1 430 Mol 2-Ethylpyridin/g-Atom Cobalt bzw. 2 592,9 kg 2-Ethylpyridin/kg Cobalt.

Nr. 20 Trimethylsilyl-$\eta^5$-Indenyl-Cobalt-Cycloocta-(1,5)-dien

Aus Pentan gewinnt man durch Abdampfen ein tiefrotes Öl, das sich bei einer Badtemperatur von 130-190 °C ($1{,}3 \cdot 10^{-3}$ mbar ; $10^{-3}$ Torr) destillieren läßt. Es resultiert ein tiefrotes, hochviskoses Öl.

Elementaranalyse

| | | | | |
|---|---|---|---|---|
| gefunden : | C 67,84 | H 7,66 | Co 16,55 % | Si 7,86 % |
| berechnet : | C 67,77 | H 7,68 | Co 16,63 | Si 7,92 % |

Massenspektrum :
m/e : 354 $M^-$, (100 %) ; 279 (63 %) ; 246 (25 %) ; 59 (17 %).

$^1$H-NMR ($d_8$-Toluol, 80 Mhz) :

$\delta H_1$ : 5,29 (d, J = 2,7 Hz)
$\delta H_2$ : 3,97 (d, J = 2,7 Hz)
$\delta H_3$ : 7,50 (m)
$\delta H_4$ : 7,08 (m)
$\delta H_5$ : 3,50 (m)
$\delta H_6$ : 3,03 (m)
$\delta H_7$ : 2,08 (m)
$\delta H_8$ : 1,35 (m)
$\delta H_9$ : 0,34 (s)

$^{13}$C-NMR ($d_8$-Toluol) :

$\delta C_{1\cdot2}$ : 68,43
$\delta C_{2\cdot1}$ : 66,09
$\delta C_{3\cdot4}$ : 31,55
$\delta C_{4\cdot3}$ : 31,29
$\delta C_5$ : 78,78

(Fortsetzung)

$^{13}C$-NMR ($d_8$-Toluol) :

| | | |
|---|---|---|
| $\delta C_6$ | : | 94,51 |
| $\delta C_7$ | : | 78,87 (s) |
| $\delta C_{8\cdot 9}$ | : | 108,81 (s) |
| $\delta C_{9\cdot 8}$ | : | 108,53 (s) |
| $\delta C_{10\cdot 13}$ | : | 125,04 |
| $\delta C_{13\cdot 10}$ | : | 124,72 |
| $\delta C_{11\cdot 12}$ | : | 123,61 |
| $\delta C_{12\cdot 11}$ | : | 123,52 |
| $\delta C_{14}$ | : | — 0,13 |

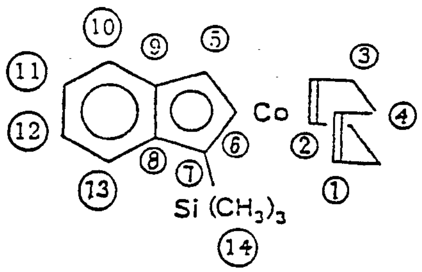

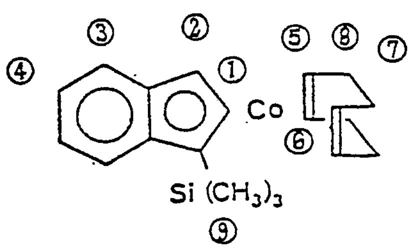

Anwendung als Katalysator

0,0746 g (0,2108 mMol) Trimethylsilylindenyl-Cobalt-Cycloocta-(1,5)-dien löst man in 117,6 g (2,868 Mol) Acetonitril und saugt diese Lösung bei Raumtemperatur in einen 500 ml Edelstahlautoklaven mit innen angebrachter Schlangenkühlung. Man sättigt das Acetonitril bei 15 bar mit Acetylen, wodurch ca. 52,0 g (2,000 Mol) Acetylen zugegeben werden.

Innerhalb von 20 min wird auf 90 °C aufgeheizt, wobei der Druck auf 34 bar ansteigt. Erhöhung der Reaktionstemperatur auf 170 °C innerhalb von 82 min läßt den Druck auf den Maximalwert von 38 bar ansteigen. Nach insgesamt 142 min Reaktionszeit bringt man das Druckgefäß binnen 45 min durch Innenkühlung mit Wasser auf 21 °C.

146,3 g Rohprodukt werden aus dem Autoklaven ausgefüllt und die flüchtigen Bestandteile bei ($1{,}3 \cdot 10^{-3}$ mbar ; $10^{-3}$ Torr) abkondensiert, wobei 0,4 g Rückstand hinterbleiben. Das Kondensat 145,6 g enthält laut gaschromatographischer Analyse 87,31 g (2,130 Mol) Acetonitril, 46,69 g (0,502 Mol) 2-Picolin und 7,38 g (0,095 Mol) Benzol, entsprechend einer 34,8 %igen 2-Picolin-Lösung in Acetonitril. Das molare Verhältnis 2-Picolin/Benzol beträgt dabei 5,3 : 1 und die Ausbeute an 2-Picolin bezogen auf umgesetztes Acetonitril : 67,9 %.

Umsatz : 27,8 % Acetonitril

Katalysatorausnutzung : 2 381 Mol 2-Picolin/g-Atom Cobalt bzw. 3 754,1 kg 2-Picolin/kg Cobalt.

Nr. 21 $\eta^5$-Indenyl-Cobalt-Norbornadien

Aus Pentan durch Abkühlen auf — 80 °C. Vom auskristallisierten Komplex drückt man die überstehende Mutterlauge ab und wäscht die Kristalle 2 mal mit je 50 ml — 80 °C kaltem Pentan. Nach Trocknen im Vakuum (0,13 mbar ; $10^{-1}$ Torr) erhält man rotbraune Nadeln vom Smp. 58 °C.

Elementaranalyse :

| | | | |
|---|---|---|---|
| gefunden : | C 72,32 | H 5,52 | Co 22,12 % |
| berechnet : | C 72,19 | H 5,64 | Co 22,15 % |

$^{1}$N-NMR ($D_8$-Toluol, 80 MHz) :

$\delta H_1$ : 7,14 (s)
$\delta H_2$ : 5,75 (t, J = 2,0 Hz)
$\delta H_3$ : 4,03 (d, J = 2,0 Hz)
$\delta H_4$ : 2,81 (m)
$\delta H_5$ : 2,64 (m)
$\delta H_6$ : 0,64 (m)

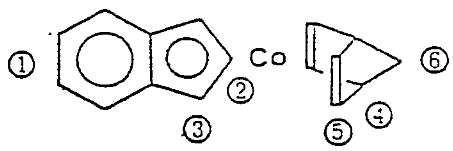

Massenspektrum :
m/e : 266 ($M^-$, 95 %) ; 239 (24 %) ; 174 (50 %) ; 150 (97 %) ; 115 (100 %) ; 59 (40 %).

Beispiel 22

Regeneration des Anthracen-Katalysators

72 g (3,00 Mol) Magnesiumpulver (Korngröße < 0,15 mm) werden unter Schutzgas mit 11 g (62 mMol) Anthracen, 3 000 ml THF und 1 ml Methyljodid versetzt. Es bildet sich unter Rühren bei 23 °C eine gelbgrüne Lösung, aus der sich nach ca. 2 Stunden orange gefärbtes Magnesium-Anthracen ausscheidet. Man aktiviert nun ca. 3 Stunden diese Raktionsmischung im Ultraschallbad. Dann erwärmt man unter Rühren auf 65 °C. Nach Zugabe von 270 g (2,5 Mol) Cyclooctadien und 290 g (2,5 Mol) Inden entfernt man die Wärmequelle und dosiert innerhalb 30 Minuten 356 g (1,00 Mol) Cobalt III-acetylacetonat fest zu. Dabei färbt sich die Reaktionsmischung unter starker Wärmetönung (bis 73 °C) tiefrotbraun. Nach Abkühlen auf 23 °C filtriert man vom unlöslichen und überschüssigen Magnesium über eine G-3-Glasfritte ab und engt das klare, rotbraune Filtrat im Hochvakuum ($1,3 \cdot 10^{-3}$ mbar ; $10^{-3}$ Torr) bis zur Trockne ein (Badtemp. : max. 40 °C). Den Rückstand nimmt man in 2 × 3 000 ml heißem Toluol auf und filtriert heiß vom Unlöslichen ab. Den Filterkuchen wäscht man portionsweise mit insgesamt 500 ml heißem Toluol aus. Durch das klare, intensiv rotbraun gefärbte Filtrat leitet man Sauerstoff zur Oxidation des Komplexes und filtriert erneut heiß. Sodann chromatographiert man die Aromatenlösung bei 60 °C über eine 60 cm $Al_2O_3$-Säule der Aktivitätsstufe 4 und verdampft das Toluol im Vakuum. Man erhält durch Umkristallisieren aus Xylol 7,6 g Anthracen, entsprechend 69 % des eingesetzten Katalysators.

Massenspektrum :
(m/e : 178 ($M^+$).

Beispiel 23

$\eta^3$-Methylheptadienyl-Cobalt-Butadien

Man aktiviert gemäß Beispiel Nr. 1 2,4 g (100 mMol) Magnesiumpulver in 200 ml THF mit 1,1 g (6,2 mMol) Anthracen, kühlt das orange gefärbte Reaktionsgemisch auf — 40 °C ab, versetzt mit 200 g (3 700 mMol) flüssigem Butadien-1,3 und dosiert innerhalb 60 Minuten 13,0 g (100 mMol) festes Co(II)chlorid zu. Unter leichtem Temperaturanstieg (max. — 34 °C) verändert sich dabei die Farbe der Reaktionsmischung über grau-grün nach grau-braun. Nach Rühren bei — 40 °C über Nacht kühlt man auf — 80 °C ab und filtriert vom Unlöslichen bzw. Ausgefallenen bei — 80 °C ab. Das klare, braune Filtrat engt man im Hochvakuum ($1,3 \cdot 10^{-3}$ mbar ; $10^{-3}$ Torr) von — 80 °C bis — 30 °C ein, nimmt den Rückstand in 150-200 ml Ethanol auf und kristallisiert den Komplex bei — 90 bis — 100 °C aus. Die überstehende Mutterlauge drückt man ab und wäscht die Kristalle 2 mal mit je 20 ml — 100 °C kaltem Pentan. Nach Trocknen im Hochvakuum ($1,3 \cdot 10^{-3}$ mbar ; $10^{-3}$ Torr) bei — 30 °C erhält man 13,5 g (60,9 mMol = 60,8 %) Methylheptadienyl-Cobalt-Butadien.

Massenspektrum :
m/e : 222 ($M^-$).

Beispiel 24

Bis-cyclooctadien-(1,5)-Nickel-(0)

7,2 g (300 mMol) Magnesiumpulver werden zusammen mit 1,1 g (6,2 mMol) Anthracen (Mg : Ant = 48 : 1) evakuiert und unter Argon gesetzt. Nach dem Suspendieren in 300 ml über $LiALH_4$ getrocknetem THF wird 0,1 ml Ethylbromid zugesetzt. Nach einigen Minuten färbt sich die Lösung gelbgrün und schon bald scheidet sich orangefarbenes Magnesiumanthracen ab. Diese Reaktion ist normalerweise nach 3 Stunden beendet. Die Suspension wird auf 0 °C gekühlt und unter Rühren 61 ml (54 g-500 mMol) Cyclooctadien-(1,5), ca. 15 ml flüssiges Butadien zugehebert und 12,96 g (100 mMol) wasserfreies Nickelchlorid in fester Form zugegeben. Schon bald färbt sich die Suspension dunkel. Über

Nacht läßt man bei Eiskühlung weiterreagieren. Es wird praktisch keine Wärmetönung beobachtet.

Die inzwischen intensiv violett gefärbte Lösung wird durch Filtration durch eine D-4-Fritte und zweimaligem Nachwaschen mit je 50 ml THF vom überschüssigen Magnesium befreit (zurückgewogene Menge Mg Ca. 3,5 g) und 3 Stunden auf — 80 °C gekühlt. Die feinen gelben Kristalle werden bei — 80 °C mit Hilfe einer D-4-Mantelfritte abfiltriert und je zweimal mit wenig THF und Pentan nachgespült. Sie werden dabei vollständig von der tiefvioletten Lösung befreit und die hellgelbe Farbe des $Ni(COD)_2$ wird sichtbar. Das Produkt wird bei 23 °C 30 Minuten im Ölpumpenvakuum getrocknet und anschließend in ein geeignetes Gefäß umgefüllt. (Diese Zwischenisolierung ist nicht unbedingt erforderlich.)

Auswaage : 21,0 g

Zur Reinigung und vollständigen Abtrennung vom $NiCl_2$ wird das $Ni(COD)_2$ umkristallisiert. Dazu füllt man in eine mittelgroße D-4-Fritte um, versetzt mit ca. 200 ml 40 °C warmen Toluol und drückt die Lösung nach dem Umrühren rasch in eine bei ± 0 °C gekühlte Vorlage. Das Nachwaschen ist ggfs. ein- bis zweimal zu wiederholen, bis die Kristalle praktisch gelöst sind. Das Filtrat wird 2 Stunden bei — 80 °C gekühlt, die resultierenden Kristalle bei — 80 °C über eine D-4-Fritte abfiltriert, zweimal mit wenig Pentan gewaschen, getrocknet und umgefüllt.

Auswaage : 16,5 g feine, zitronengelbe Blättchen von $Ni(COD)_2$ = 60 % d. Th. (bez. auf $NiCl_2$)
Elementaranalyse :
gefunden : C 69,89 H 9,06 Ni 21,12 %
berechnet : C 69,86 H 8,79 Ni 21,34 %

## Beispiel 25

Bis(cyclooctadien-1,5)-Platin

Man aktiviert gemäß Beispiel Nr. 1 0,49 g (20 mMol) Mg in 50 ml THF mit 71 mg (0,4 mMol) Anthracen, erwärmt die orange gefärbte Reaktionsmischung auf 65 °C, setzt 16,2 g (150 mMol) Cyclooctadien-1,5 zu und dosiert innerhalb 20 Minuten festes Platin-II-chlorid zu. Die Reaktionsmischung färbt sich dabei unter starker Wärmetönung (bis max. 79 °C) rotbraun. Nach Abkühlen auf 23 °C engt man die Reaktionsmischung im Hochvakuum ($1,3 \cdot 10^{-3}$ mbar ; $10^{-3}$ Torr) bis zur Trockne ein und extrahiert den Rückstand 6 mal mit 100 ml Toluol. Die braune Lösung filtriert man durch eine Säule mit $Al_2O_3$ (5 cm mit 7 % $H_2O$ desaktiviert) und engt das Filtrat im Vakuum auf ca. 30 ml ein. Vom ausgefallenen hellen Niederschlag drückt man die Mutterlauge ab, wäscht mit kaltem Toluol und erhält 3,7 g (9 mMol = 45 %) Bis(cyclooctadien-1,5) Platin.

Massenspektrum :
m/e : 410 ($M^-$) ; 302.

## Beispiel 26

Bis(Cyclooctadien-1,5) Palladium

Man aktiviert gemäß Beispiel Nr. 1 0,6 g (25 mMol) Mg in 60 ml THF mit 90 mg (0,5 mMol) Anthracen und kühlt die orange gefärbte Reaktionsmischung auf — 40 °C, versetzt mit 35 ml Cyclooctadien-1,5, 1 ml Butadien-1,3 und dosiert 7,1 g (25 mMol) festes Cyclooctadien-1,5-Palladiumdichlorid innerhalb 30 Minuten zu. Nach 1 Stunde wird von der dunklen Reaktionsmischung alles Flüchtige bei — 40 °C im Hochvakuum ($1,3 \cdot 10^{-3}$ mbar ; $10^{-3}$ Torr) schnell abgezogen, der Rückstand in 100 ml Pentan (mit 5 ml Butadien versetzt) aufgenommen und durch eine — 30 °C gekühlte $Al_2O_3$-Säule (5 cm) filtriert. Nach Einengen des Filtrates fallen 1,2 g = 15 % d. Th. Produkt an, das durch weiteres Umkristallisieren gereinigt werden kann.

## Beispiel 27

Tris(butadien)molybdän

3,3 g (135,8 mMol) Magnesium werden analog Beispiel 1 in 500 ml THF mit 1,5 g (0,84 mMol) Anthracen versetzt und im Ultraschallbad aktiviert. Man kühlt die Mischung auf — 30 °C ab und hebert 23 ml flüssiges Butadien dazu. Während 30 Minuten dosiert man unter Schutzgas das hydrolyseempfindliches $MoCl_5$ (9,8 g = 35,8 mMol) zu der Mischung, was eine stark exotherme Reaktion auslöst. Das Reaktionsgemisch färbt sich in dieser Zeit bereits braun. Bei — 10 °C und ($1,3 \cdot 10^{-2}$ mbar ; $10^{-2}$ Torr) dampft man das THF ab und trocknet den festen Rückstand bei 20 °C und ($1,3 \cdot 10^{-2}$ mbar ; $10^{-2}$ Torr). Die Extraktion mit Toluol lieferte nach dem Eindampfen ein klebriges Rohprodukt. Dieses wird mit 1 000 ml Pentan nochmals ausgezogen und durch Abkühlen wiedergewonnen. Man löst erneut in 30 ml THF von 50 °C und bringt die Lösung auf — 20 °C. Zugabe von 80 ml kaltem Ether lieferte 3,7 g entsprechend 40 % d. Th. $(C_4H_6)_3Mo$.

Beispiel 28

Tetrakis-Triphenylphosphan-Palladium

Man aktiviert 0,48 g (20 mMol) Magnesiumpulver in 40 ml THF mit 0,1 g (0,56 mMol) Anthracen gemäß Beispiel Nr. 1 und erwärmt das orange gefärbte Reaktionsgemisch auf 65 °C, versetzt mit 21,0 g (80 mMol) Triphenylphosphan und dosiert innerhalb 20 Minuten 6,1 g (20 mMol) festes Pd $acac_2$ zu. Dabei fällt aus der dunkelorange gefärbten Reaktionsmischung unter Wärmetönung bis 69 °C der Komplex aus. Nach Abkühlen auf 23 °C filtriert man die Kristalle über eine G-3-Fritte ab, wäscht mit 30 ml Pentan und erhält nach Trocknen in Vakuum (0,13 mbar ; $10^{-1}$ Torr) 20,6 g (17,9 mMol) = 89,5 % d. Th.) gelbes kristallines Produkt vom Smp. 116 °C.

Beispiel 29

Reduktion von Pd $acac_2$ mit Mg ohne Aktivator

240 mg (10 mMol) Magnesiumpulver werden in 40 ml THF aufgeschlämmt, 2 Stunden im Ultraschallbad behandelt und mit 10,5 g (40 mMol) Triphenylphosphan versetzt. Man erwärmt auf 65 °C und dosiert innerhalb 10 Minuten 3,05 g (10 mMol) festes Palladium-II-acetylacetonat zu, wobei sich das Reaktionsgemisch hellbraun färbt. Nach 6 Stunden Rühren bei 65 °C scheidet sich eine hellgelbe Verbindung aus. Nach Abkühlen filtriert man den Niederschlag ab, wäscht ihn mit 20 ml Ether und erhält 5,9 g einer hellgelben Triphenylphosphin-Palladium-acetylacetonat-Verbindung.

Elementaranalyse :

gefunden : Pd : 13,00 % ; P 9,40 %

Infrarotspektrum :

Acetylacetonato-Banden : 1655 ; 1550 ; 1350 ; 1232 $cm^{-1}$

Triphenylphosphinbanden : 1480 ; 1430 ; 1180 ; 1150 ; 1090 ; 1020 ; 995 ; 510 $cm^{-1}$.

Beispiel 30

Bis(Triorthotolylphosphit)Nickel-Ethylen

Man aktiviert wie in Beispiel Nr. 12,4 g (100 mMol) Magnesiumpulver in 300 ml THF mit 1,1 g (6,2 mMol) Anthracen und kühlt das orange gefärbte Reaktionsgemisch auf 0 °C. Nach Zugabe von 70,5 g (200 mMol) Triorthotolylphosphit leitet man 10 Minuten Ethylengas durch die Mischung und dosiert 25,7 g (100 mMol) Nickel(II)-acetylacetonat unter permanentem Ethylengasstrom innerhalb 10 Minuten fest zu. Dabei steigt die Innentemperatur trotz Außenkühlung mit Eis auf 15 °C an. Man läßt drei Stunden unter Ethylenstrom nachreagieren und filtriert von evtl. Unlöslichem über eine G-3-Fritte ab. Das gelbbraune Filtrat wird im Vakuum (0,13 mbar ; $10^{-1}$ Torr) bis zur Trockne eingeengt und der glasig-spröde Rückstand in 300 ml Pentan und 100 ml Toluol aufgenommen. Man filtriert von Unlöslichem ab und kristallisiert das Produkt durch Zugabe von 100 ml Methanol aus. Durch Abkühlen auf — 20 °C vervollständigt man die Kristallisation.

Ausbeute :

51,5 g = 65 % d. Th. gelbe Kristalle vom Schmelz- bzw. Zersetzungspunkt 118 °C.

Beispiel 31

Tris(Triortho-Tolylphosphit)Nickel

Man aktiviert gemäß Beispiel Nr. 11,44 g (60 mMol) Magnesiumpulver in 180 ml THF mit 0,66 g (3,7 mMol) Anthracen. Man versetzt bei 23 °C mit 70,5 g (200 mMol) Triortho-Tolylphosphit und dosiert innerhalb 20 Minuten 15,4 g (60 mMol) Nickel(II)-acetylacetonat in fester Form zu. Das Reaktionsgemisch wird im Vakuum ($1,3 \cdot 10^{-2}$ mbar ; $10^{-2}$ Torr) vom Flüchtigen befreit und der Rückstand in 200 ml Pentan und 70 ml Toluol aufgenommen. Man filtriert von Unlöslichem ab, versetzt das Filtrat mit 150 ml Methanol und kühlt auf — 20 °C. Hierbei scheidet sich der Komplex in hellroten Kristallen ab.

Ausbeute : 50,2 g = 75 % d. Th.

Beispiel 32

Pentamethyl-$\eta^5$-cyclopentadienyl-cobalt-bis-ethylen

48,2 mg (2,01 mMol) feines Magnesiumpulver wird analog Beispiel Nr. 1 mit 8,3 mg (0,047 mMol) Anthracen in 8 ml THF versetzt und im Ultraschallbad zusätzlich aktiviert. Man leitet bei 0 °C gasförmig Ethylen durch die Lösung und gibt 893,8 mg entsprechend 1,99 mMol $[(Co(C_5Me_5)I_2)_2]$ auf einmal hinzu. Die anfangs grün-schwarze Suspension geht in eine orange-braune Lösung über, die bei 0,13 mbar (0,1

Torr) bis 25 °C abgedampft wird. Man nimmt in 8 ml Pentan auf, filtriert und kühlt auf —80 °C. Nach einiger Zeit fallen 373,1 mg orange gefärbte Kristalle $Me_5CpCo(C_2H_4)_2$ an entsprechend 75 % d. Th.

Beispiel 33

Bis-(Pentamethyl-$\eta^5$-Cyclopentadienyl)Zirkondicarbonyl

72 mg (2,96 mMol) feines Magnesiumpulver wird analog Beispiel 1 mit 10,1 mg (0,057 mMol) Anthracen in 25 ml THF versetzt und im Ultraschallbad zusätzlich 5 Stunden aktiviert. Anschließend leitet man CO ein, wobei für gute Durchmischung gesorgt werden muß. Man gibt 869,2 mg entsprechend 2,01 mMol $(\eta^5C_5Me_5)_2ZrCl_2$ zu und läßt 2 Stunden reagieren. Die dunkelrote Lösung wird zur Trockne eingedampft und der Rückstand in 50 ml Pentan aufgenommen. Man filtriert von Schwebeteilchen ; aus der auf 20 ml eingeengten Lösung erhält man bei —30 °C 696,1 mg entsprechend 83 % d. Th. $(\eta^5C_5Me_5)_2Zr(CO)_2$ als schwarze Nadeln.

Beispiel 34

($\mu$-dichloro) (Biscyclopentadienyl-Titan)-fulven

10,94 g (450 mMol) feines Magnesiumpulver in 300 ml THF werden wie in Beispiel Nr. 1 mit 1,1 g (6,2 mMol) Anthracen sowie 42,7 g Cyclopentadien versetzt, mit 0,1 ml Methyljodid aktiviert und 3 Stunden im Ultraschallbad behandelt. Man gibt tropfenweise 56,9 g (33 ml = 300 mMol)$TiCl_4$ zu, was eine sehr heftige Reaktion und Ansteigen der Temperatur auf 69 °C auslöst. Man läßt nach Ende der Zugabe auf 23 °C abkühlen und erhält binnen 12 Stunden 14,8 g einer scharlachroten Verbindung, die durch Filtration isoliert wurde. Aus dem Filtrat gewinnt man durch Einengen weitere 69,1 g.

Gesamtausbeute :

83,9 g $C_5H_4$-$C_5H_4Ti_2(C_5H_5)_2Cl_2$ entsprechend 179,9 mMol = 66 % d. Th.

Elementaranalyse :

gefunden : C : 40,05 % ; H : 5,75 % ; Ti : 7,26 % ; Cl : 32,21 %

Massenspektrum :

m/e : 424 ($M^-$).

Beispiel 35

$Cp_2TiCl_2$

Aus einem Ansatz analog Beispiel 34 mit 1,2 g (50 mMol) Magnesiumpulver, 0,2 g (1 mMol) Anthracen in 50 ml THF sowie 8,4 g (128 mMol) Cyclopentadien und 16,7 g (50 mMol) $TiCl_4 \cdot 2THF$ erhält man durch Eindampfen des Reaktionsgemisches zur Trockne und Extraktion mit $CH_2Cl_2$ sowie nachfolgender Kristallisation 6 g blaßrotes (24,2 mMol) $Cp_2TiCl_2$ entsprechend 48,4 % d. TH.

Elementaranalyse :

gefunden : C : 45,00 % ; H : 4,19 % ; Ti : 18,59 % ; Cl : 32,5 %

Massenspektrum :

m/e : 248 ($M^-$).

Beispiel 36

Bis-$\eta^5$-Methylcyclopentadienyl-Titan-Dichlorid

1,2 g (50 mMol) Magnesiumpulver werden in 50 ml THF analog Beispiel Nr. 1 mit 0,18 g (1 mMol) Anthracen versetzt und im Ultraschallbad zusätzlich 3 Stunden aktiviert.

Anschließend erwärmt man auf 65 °C, versetzt mit 8 g (100 mMol) 1-Methylcyclopentadien und dosiert innerhalb 10 Minuten 16,7 g (50 mMol) festes Titan(IV) chlorid · 2 THF zu. Unter heftiger Wärmetönung bis 70 °C verfärbt sich die Reaktionsmischung über graubraun nach scharlachrot. Nach Abkühlen auf 23 °C engt man im Vakuum ($1{,}3 \cdot 10^{-2}$ mbar ; $10^{-2}$ Torr) bis zur Trockne ein und extrahiert den Rückstand mit Chloroform. Daraus erhält man 5,92 g (21,46 mMol = 43 %) gelbrotes Produkt.

Beispiel 37

$\eta^5$-($\alpha$-Methylindenyl)-Cobalt-Cyclooctadien-1,5

7,2 g (300 mMol) Magnesiumpulver (Korngröße < 0,15 mm) werden unter Schutzgas mit 1,1 g (6,2 mMol) Anthracen, 300 ml THF und 0,1 ml Methyljodid versetzt. Es bildet sich unter Rühren bei Raumtemperatur eine gelbgrüne Lösung, aus der sich nach 1-2 Stunden orange gefärbtes Magnesium-

Anthracen abscheidet. Anschließend aktiviert man die Reaktionsmischung ca. 1 Stunde im Ultraschallbad und erwärmt unter Rühren auf 65 °C. Nach Zugabe von 27,32 g (253 mMol) COD-1,5 und 13,84 g (107 mMol) α-Methylinden entfernt man die Wärmequelle und dosiert innerhalb von 30 Minuten 35,61 g (100 mMol) Cobalt(III)acetylacetonat portionsweise zu. Dabei verfärbt sich die Reaktionslösung unter Selbsterwärmung bis zum heftigen Rückfluß (72 °C) schwarzrot. Nach Abkühlen auf Raumtemperatur läßt man über Nacht rühren (ca. 16 Stunden), filtriert von nichtreagiertem Magnesium und Unlöslichem über eine G-3-Glasfritte ab und engt das Filtrat im Hochvakuum ($10^{-3}$ mbar) bis zur Trockene ein. Den schwarzbraunen Rückstand nimmt man in 400 ml Pentan auf und filtriert erneut von Unlöslichem über eine G-3-Glasfritte ab. Den Filterkuchen wäscht man portionsweise mit insgesamt 150 ml Pentan und engt das rotbraune Filtrat bis auf 100 ml ein. Anschließend kristallisiert man durch langsames Abkühlen auf — 80 °C den Komplex aus. Man drückt die überstehende Mutterlauge ab, wäscht 2 mal mit je 50 ml — 80 °C kaltem Pentan und trocknet die Kristalle im Hochvakuum ($10^{-3}$ mbar).

Ausbeute :
14,3 g = 48,3 mMol = 48,3 %

$\eta^5$-(α-Methylindenyl)-Cobalt-Cyclooctadien-1,5

Smp. : 72-73 °C
Elementaranalyse :
ber. : C 72,97 H 7,09 Co 19,93 %
gef. : C 72,92 H 7,14 Co 19,98 %
IR-Analyse :
2 820-3 000 ; 1 468 ; 1 452 ; 1 424 ; 1 370 ; 1 335 ; 1 318 ; 1 205 ; 845 ; 810 $cm^{-1}$.

$^1H$-NMR ($d_8$-Toluol) :

$\delta H_1$ : 5,59 ppm (d, J = 2,8 Hz)
$\delta H_2$ : 3,82 ppm (d, J = 2,8 Hz)
$\delta H_3$ : 7,20 ppm (m)
$\delta H_4$ : 1,30 ppm (s)
$\delta H_5$ : 3,21 ppm (m)
$\delta H_6$ : 2,12 ppm (m)
$\delta H_7$ : 1,50 ppm (m)

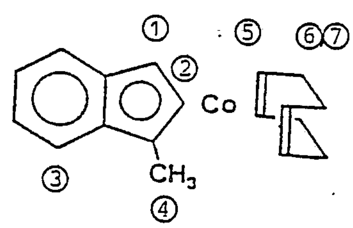

$^{13}C$-NMR ($d_8$-Toluol) :

$\delta C_{1,4}$ : 69,71 ppm
$\delta C_2$ : 31,87 ppm
$\delta C_3$ : 30,81 ppm
$\delta C_{4,1}$ : 67,67 ppm
$\delta C_5$ : 84,97 ppm
$\delta C_6$ : 89,82 ppm
$\delta C_7$ : 73,86 ppm
$\delta C_8$ : 105,59 ppm
$\delta C_9$ : 105,44 ppm
$\delta C_{10/13}$ : 121,06 ppm
$\delta C_{11/12}$ : 124,04 ppm
$\delta C_{13/10}$ : 123,40 ppm
$\delta C_{14}$ : 9,81 ppm

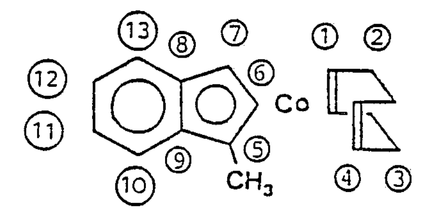

Massenspektrum :
m/z : 296 (100 %) ; 266 (44 %) ; 188 (86 %) ; 129 (42 %) ; 128 (60 %) ; 113 (38 %) ; 59 (89 %).

## Beispiel 38

Zu 2.43 g (0.10 mol) Magnesiumpulver mit einer durchschnittlichen Korngröße bis 0,3 mm (50 mesh) in 150 ml THF werden 0.36 g (2.0 mmol) Anthracen und 0.1 ml Ethylbromid zugegeben und die Suspension bei Raumtemperatur bis zum Ausfallen des orangefarbenen Niederschlages von Magnesiumanthracen (ca. 2 h) gerührt. Die Suspension wird bei 0 °C mit 26 ml (0.3 mol) flüssigem Butadien und anschließend innerhalb von 30 min. und unter Rührend mit 13.0 g (0.10 mol) wasserfreiem $NiCl_2$ versetzt. Nach 20 h Rühren bei 0 °C wird der Ansatz auf — 78 °C gekühlt, bei gleichbleibender Temperatur vom $MgCl_2$ und nicht umgesetzten Magnesium und $NiCl_2$ abfiltriert und der Filterkuchen mit kaltem THF nachgewaschen. Das tiefrotgefärbte Filtrat enthält 82.5 % des eingesetzten Nickels in löslicher Form. Eine 10 ml Probe von insgesamt 275 ml des Filtrats nimmt bei der Hydrierung 328 ml $H_2$ (25 °C, 1 bar) auf, wobei metallisches Nickel quantitativ ausgeschieden wird. Nach Entfernen von THF und Destillation im Hochvakuum erhält man 0.62 g gesättigter Kohlenwasserstoffe folgender Zusammensetzung (in Gew.%, nach GC-Analyse) : n-Dodekan 76.8, Cyclododekan 10.3, n-Oktan 3.1 und n-Hexadekan 0.5 % (Rest unbekannte Verbindungen). Die n-Dodekanmenge entspricht einer Ausbeute des $\eta^3$, $\eta^2$, $\eta^3$-Dodeka-2,6,10-trien-1,13-diyl-nickels (I) (Bogdanović, Heimbach, Kröner, Wilke, Hoffmann & Brandt, Liebigs Ann. Chem.

727, 143 (1969)) von 77 % (bez. auf einges. $NiCl_2$) ; das Molverhältnis Ni : n-Dodekan beträgt 1.00 : 0.93 (theor. 1 : 1).

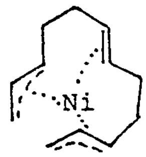

(I)

Zum Vergleich wurde ein Versuch in gleicher Weise und mit gleichen Stoffmengen, jedoch ohne Einsatz von Anthracen durchgeführt. Dabei wurden nach der Filtration bei tiefer Temperatur nur 37 % des eingesetzten Nickels in Lösung wiedergefunden.

Beispiel 39

$\eta^6$-Phenylborinato-Cobalt-Cyclooctadien-1,5

146 mg (6 mMol) Magnesiumpulver (Korngröße < 0,15 mm) werden unter Schutzgas mit 24 mg (0,13 mMol) Anthracen, 6 ml THF und einem Tropfen Methyljodid versetzt. Es bildet sich unter Rühren bei RT eine gelbgrüne Lösung, aus der sich in ca. 1-2 Stunden orange gefärbtes Magnesium-Anthracen abscheidet. Man aktiviert ca. 3 Stunden die Reaktionsmischung im Ultraschallbad (Sonorex RK 514 der Firma Bandelin, HF-Dauerspitzenleistung 400 Watt, 35 kHz) und erwärmt unter Rühren auf 60 °C. Nach Zugabe von 550 mg (5,09 mMol) Cyclooctadien-1,5 und 350 mg (2,27 mMol) 1-Phenylboracyclohexadien-2,5 in 4 ml THF entfernt man die Wärmequelle und dosiert innerhalb 3 Minuten 710 mg (1,99 mMol) festes Cobalt (III)acetylacetonat zu. Dabei verfärbt sich die Reaktionsmischung unter Selbsterwärmung bis zum heftigen Rückfluß (73 °C) tief orangebraun. Nach Abkühlen auf RT zieht man im Vakuum ($10^{-3}$ mbar) alles Flüchtige ab, nimmt den spröden, braunen Rückstand in ca. 80 ml Pentan auf und filtriert von Unlöslichem über eine G-3-Fritte ab. Aus dem klaren orange gefärbten Filtrat kristallisiert man durch langsames Abkühlen auf — 80 °C den Komplex aus, trennt von der überstehenden Mutterlauge ab und trocknet die Kristalle im Vakuum ($10^{-1}$ mbar).

Ausbeute :

270 mg = 0,84 mMol = 42,2 %

$\eta^6$-Phenylborinato-Cobalt-Cyclooctadien-1,5

Smp : 169 °C (Lit. : 169 °C)

Literatur : G.E. Herberich, W. Koch und H. Lueken, J. Organomet. Chem. 160 (1978) 17-23.

Beispiel 40

Bis-$\eta^5$-Cyclopentadienyl-Vanadium

7,2 g (300 mMol) Magnesiumpulver (Korngröße < 0,15 mm) werden unter Schutzgas mit 1,1 g (6,2 mMol) Anthracen, 300 ml THF und 0,1 ml Methyljodid versetzt. Es bildet sich unter Rühren bei RT eine gelbgrüne Lösung, aus der sich in ca. 1-2 Stunden orange gefärbtes Magnesium-Anthracen abscheidet. Man aktiviert ca. 3 Stunden die Reaktionsmischung im Ultraschallbad (Sonorex RK 514 der Firma Bandelin, HF-Dauerspitzenleistung, 400 Watt, 35 kHz) und erwärmt unter Rühren auf 65 °C. Nach Zugabe von 26,4 g (400 mMol) monomeren Cyclopentadien dosiert man innerhalb 10 Minuten 15,7 g (100 mMol) festes Vanadium(III)chlorid zu. Man erhitzt weitere 4 1/2 Stunden auf 60 °C und filtriert nach Abkühlen auf RT von Unlöslichem über eine G-3-Glasfritte ab. Das tiefviolette Filtrat engt man im Vakuum ($10^{-1}$ mbar) bis zur Trockene ein und nimmt den schwarzvioletten Rückstand in ca. 150 ml Pentan auf. Man filtriert erneut von Unlöslichem über eine G-3-Glasfritte ab, kühlt das klare tiefviolette Filtrat langsam auf — 80 °C und läßt den Komplex über Nacht auskristallisieren. Die überstehende Mutterlauge drückt man ab, wäscht die Kristalle 2 mal mit je 30 bis 40 ml — 80 °C kaltem Pentan und trocknet im Vakuum ($10^{-1}$ mbar) bei RT.

Ausbeute :

3,4 g = 18,8 mMol = 18,8 %

Bis-$\eta^5$-Cyclopentadienyl-Vanadium

Smp. : 166 °C (Lit. : 167-168 °C)

Literatur : E. O. Fischer, W. Hafner, Z. Naturforsch. 9b (1954) 503-504.

Beispiel 41

$\eta^5$-Cyclopentadienyl-Cobalt-Cyclooctadien-1,5 (zweistufig)

1. Stufe

7,2 g (300 mMol) Magnesiumpulver (Korngröße < 0,15 mm) werden unter Schutzgas mit 1,1 g (6,2 mMol) Anthracen, 150 ml THF und 0,1 ml Methyljodid versetzt. Es bildet sich unter Rühren bei RT eine gelbgrüne Lösung, aus der sich in ca. 1-2 Stunden orange gefärbtes Magnesium-Anthracen abscheidet. Man kühlt die Reaktionsmischung auf — 25 °C, versetzt mit 32,4 g (300 mMol) Cyclooctadien-1,5 und dosiert innerhalb 3 Stunden 20 Minuten 21,9 g (100 mMol) Cobalt(II)bromid fest zu. Dabei verfärbt sich die Reaktionsmischung von grün nach dunkelgraugrün. Man läßt über Nacht bei — 30 °C (16 Stunden) rühren und versetzt die grünschwarze heterogene Reaktionsmischung mit 100 ml — 30 °C kaltem Toluol und filtriert bei — 30 °C von Unlöslichem über eine G-3-Glasfritte mit Kühlmantel ab. Den graugrünen Frittenrückstand trocknet man 3 Stunden im Hochvakuum ($10^{-3}$ mbar) und extrahiert ihn mit 400 ml 80 °C heißem Toluol. Man filtriert in der Hitze (80 °C) von Ungelöstem ab. Durch langsames Abkühlen auf RT scheidet sich aus dem klaren dunkelgrünen Filtrat ein grünschwarzer Festkörper ab. Nach Abdrücken der überstehenden Mutterlauge und Trocknen im Hochvakuum ($10^{-3}$ mbar) erhält man 2,8 g eines schwarzgrünen Feststoffes.

Elementaranalyse :
gef. : C = 32,56 % ; H = 4,80 % ; Co = 7,26 % ; Mg = 6,83 % ; Br = 40,37 %.

2. Stufe

Man löst 700 mg (≙ 0,83 mMol Cobalt) des oben beschriebenen grünschwarzen Feststoffes in 50 ml THF und dosiert 560 mg (3,15 mMol) Cyclopentadienylnatrium-Dimethoxyethan-Addukt bei RT fest zu. Man erhitzt 6 Stunden auf 60 °C, wobei eine braune Lösung und eine heller Niederschlag resultiert. Man filtriert vom Niederschlag über eine G-4-Glasfritte ab und engt das braune Filtrat im Hochvakuum ($10^{-3}$ mbar) bis zur Trockene ein. Den Rückstand nimmt man in ca. 100 ml Pentan auf, filtriert von Unlöslichem ab und engt das Filtrat auf ca. 30 ml ein. Man versetzt mit 20 ml sauerstofffreiem Wasser und rührt ca. 1 Stunde kräftig. Anschließend isoliert man die orange gefärbte organische Phase und trennt sie an einer ca. 30 cm langen $Al_2O_3$-Säule (Aktivitätsstufe II) adsorptionschromatographisch auf. Der orange gefärbte Hauptlauf wird auf ca. 10 ml eingeengt und der Komplex durch Abkühlen auf — 80 °C auskristallisiert. Die überstehende Mutterlauge drückt man ab und trocknet die orangebraunen Kristalle im Vakuum ($10^{-1}$ mbar).

Ausbeute :
19 mg = 0,082 mMol = 9,9 %

$\eta^5$-Cyclopentadienyl-Cobalt-Cyclooctadien-1,5

Smp. : 102 °C (Lit. : 103 °C)
Literatur : R.B. King, P.M. Treichel und F.G.A. Stone, J. Am. Chem. Soc. 83 (1981) 3593-3597.

Beispiel 42

$\eta^5$-Cyclopentadienyl-Cobalt-Cyclooctadien-1,5 (zweistufig)

1. Stufe

7,2 g (300 mMol) Magnesiumpulver (Korngröße < 0,15 mm) werden unter Schutzgas mit 1,1 g (6,2 mMol) Anthracen, 150 ml THF und 0,1 ml Methyljodid versetzt. Es bildet sich unter Rühren bei RT eine gelbgrüne Lösung, aus der sich in ca. 1-2 Stunden orange gefärbtes Magnesium-Anthracen abscheidet. Man kühlt die Reaktionsmischung auf — 25 °C, versetzt mit 32,4 g (300 mMol) Cyclooctadien-1,5 und dosiert innerhalb 3 Stunden 13,0 g (100 mMol) Cobalt(II)chlorid fest zu. Dabei verfärbt sich die Reaktionsmischung von blau nach graugrün. Man läßt 48 Stunden bei — 30 °C rühren und versetzt die dunkelgraugrüne heterogene Reaktionsmischung mit 100 ml — 30 °C kaltem Toluol und filtriert bei — 30 °C von Unlöslichem über eine G-3-Glasfritte mit Kühlmantel ab. Den graugrünen Frittenrückstand wäscht man 2 mal mit je 200 ml Pentan und trocknet ihn 3 Stunden im Hochvakuum ($10^{-3}$ mbar). Das graugrüne Pulver extrahiert man mit 500 ml 80-90 °C heißem Toluol und filtriert in der Hitze (80 °C) von Ungelöstem ab. Aus dem klaren dunkelgrünen Filtrat scheidet sich durch langsames Abkühlen auf RT ein grünschwarzer Festkörper ab. Nach Abdrücken der überstehenden Mutterlauge und Trocknen im Hochvakuum ($10^{-3}$ mbar) erhält man 19,5 g eines grünschwarzen, glänzenden Feststoffes.

Elementaranalyse :
gef. : C = 54,76 % ; H = 8,28 % ; Co = 7,27 % ; Mg = 5,71 % ; Cl = 13,11 %.

2. Stufe

Man löst 1,2 (≙ 1,5 mMol Cobalt) des oben beschriebenen grünschwarzen Feststoffes in 75 ml THF und dosiert 5,2 g (29 mMol) Cyclopentadienylnatrium-Dimethoxyethan-Addukt bei RT fest zu. Man erhitzt über Nacht (ca. 16 Stunden) auf 70 °C, wobei eine braune Lösung und eine heller voluminöser Niederschlag entsteht. Man filtriert vom Niederschlag über eine G-4-Glasfritte ab und engt das braune Filtrat im Hochvakuum ($10^{-3}$ mbar) ein. Den öligen Rückstand nimmt man in 100 ml Pentan auf, filtriert von Unlöslichem ab und trennt das klare orange gefärbte Filtrat an einer ca. 30 cm langen $Al_2O_3$-Säule (Aktivitätsstufe II) adsorptionschromatographisch auf. Den orange-gelb gefärbten Hauptlauf engt man auf ca. 20 ml ein und kristallisiert den Komplex durch Abkühlen auf —80 °C aus. Die überstehende Mutterlauge drückt man ab und trocknet die orangebraunen Kristalle im Vakuum ($10^{-1}$ mbar).

Ausbeute :

27 mg = 0,12 mMol = 8 %

$\eta^5$-Cyclopentadienyl-Cobalt-Cyclooctadien-1,5

Smp. : 102-103 °C (Lit. : 103 °C)

Literatur : R.B. King, P.M. Treichel und F.G.A. Stone, J. Am. Chem. Soc. 83 (1981) 3593-3597.

## Beispiele 43 bis 46

Die Verwendung von durch substituierte Anthracene aktiviertem Magnesium wird durch 4 Beispiele für die Herstellung von $\eta^5$-Indenylcobalt-cycloocta-(1,5)-dien erläutert.

## Beispiel 43

Es wird nach der Arbeitsweise von Beispiel 11 verfahren, jedoch mit der Abweichung, daß 6 mmol 2-Methylanthracen an Stelle von Anthracen eingesetzt werden. Ausbeute : 20,3 g (20,3 mmol = 72 %).

## Beispiel 44

Es wird nach der Arbeitsweise von Beispiel 11 verfahren, jedoch mit der Abweichung, daß 6 mmol 9-Methylanthracen an Stelle von Anthracen eingesetzt werden. Ausbeute : 18,3 g (18,3 mmol = 65 %).

## Beispiel 45

Es wird nach der Arbeitsweise von Beispiel 11 verfahren, jedoch mit der Abweichung, daß 6 mmol 1,4-Dimethylanthracen an Stelle von Anthracen eingesetzt werden. Ausbeute : 18,9 g (18,9 mmol = 67 %).

## Beispiel 46

Es wird nach der Arbeitsweise von Beispiel 11 verfahren, jedoch mit der Abweichung, daß 6 mmol 9,10-Diphenylanthracen an Stelle von Anthracen eingesetzt werden. Ausbeute : 16,1 g (16,1 mmol = 57 %).

**Patentansprüche**

1. Verfahren zur Herstellung von Komplexverbindungen der Übergangsmetalle, dadurch gekennzeichnet, daß man Übergangsmetallsalze in Gegenwart komplexbildender Liganden mit Magnesium umsetzt, dem eine katalytische Menge Anthracen und/oder Magnesiumanthracen als Aktivator zugesetzt ist.

2. Verfahren nach Anspruch 1, dadurch gekennzeichnet, daß man das Magnesium im Ultraschallbad mit einer katalytischen Menge Anthracen und/oder Magnesiumanthracen behandelt.

3. Verfahren nach Anspruch 1 und 2, dadurch gekennzeichnet, daß man als Übergangsmetalle diejenigen der Gruppen IVB bis VIIIB des Periodensystems der Elemente einsetzt.

4. Verfahren nach Anspruch 1 bis 3, dadurch gekennzeichnet, daß man als komplexbildende Liganden elektronenabgebende Verbindungen, die mit Übergangsmetallen Durchdringungskomplexe bilden, einsetzt.

5. Verfahren nach Anspruch 1 bis 4, dadurch gekennzeichnet, daß man als komplexbildende Liganden Verbindungen mit C-C-Mehrfachverbindungen wie Olefine, substituierte Olefine, cyclische Olefine, konjugierte Olefinsysteme, Mehrfacholefine, Alkine und Cycloalkine einsetzt.

6. Verfahren nach Anspruch 1 bis 4, dadurch gekennzeichnet, daß man als komplexbildende Liganden solche mit freien Elektronenpaaren wie Phosphane, Phosphite, Arsane, Triorganylantimonverbindungen, Pyridine, Dipyridine und Kohlenmonoxid einsetzt.

7. Verfahren nach Anspruch 1 bis 5 zur Herstellung von Cyclopentadienyl-Verbindungen der

Übergangsmetalle, dadurch gekennzeichnet, daß man Cyclopentadien und/oder Cyclopentadienderivate als Komplexbildner einsetzt.

8. Verfahren nach Anspruch 1 bis 5 und 7 zur Herstellung von gegebenenfalls substituierten Cyclopentadienyl-Eisenverbindungen (Ferrocenen), dadurch gekennzeichnet, daß man Eisensalze in Gegenwart von Cyclopentadien und/oder Cyclopentadienderivaten mit dem aktivierten Magnesium umsetzt.

9. Verfahren nach Anspruch 1 bis 5 und 7 zur Herstellung von Cyclopentadienyl-Cobalt-Komplexen (Cobaltocenen), dadurch gekennzeichnet, daß man Cobaltsalze in Gegenwart von Cyclopentadien und/oder Cyclopentadienderivaten mit dem aktivierten Magnesium umsetzt, wobei das molare Verhältnis von Komplexbildner zu Cobalt $\leq 3$ beträgt.

10. Verfahren nach Anspruch 1 bis 5 zur Herstellung von Cyclopentadienyl-Cobalt-Olefin-Halbsandwichverbindungen, dadurch gekennzeichnet, daß man Cyclopentadien bzw. Derivate desselben und ein weiteres Olefin als Komplexbildner einsetzt, in deren Gegenwart Cobaltsalze mit dem aktivierten Magnesium umgesetzt werden.

11. Verfahren nach Anspruch 1 bis 5 und 7 zur Herstellung von Cyclopentadienyl-Cobalt-Cyclopentadien-Verbindungen, dadurch gekennzeichnet, daß man Cobaltsalze in Gegenwart von Cyclopentadien und/oder Cyclopentadienderivaten mit dem aktivierten Magnesium umsetzt, wobei das molare Verhältnis von Komplexbildner zu Cobalt $\geq 4$ beträgt.

12. Verfahren nach Anspruch 1 bis 5 und 7 zur Herstellung von gegebenenfalls substituierten Cyclopentadienyl-Cobalt-Cycloocta(1,5)dien-Verbindungen, dadurch gekennzeichnet, daß man Cyclopentadien bzw. Derivate desselben und Cyclo-(1,5)-octadien gemeinsam als Komplexbildner am Cobalt einsetzt.

13. Verfahren nach Anspruch 1 bis 6 zur Herstellung von $\eta^3$-Allylkomplexen des Nickels und Cobalts, dadurch gekennzeichnet, daß man Salze dieser Übergangsmetalle in Gegenwart von (1,3)-Dienen und gegebenenfalls weiterer Komplexbildner mit dem aktivierten Magnesium umsetzt.

14. Verfahren nach Anspruch 1 bis 5 zur Herstellung von 5-Methyl-$\eta^3$-Heptadienylcobaltbutadien, dadurch gekennzeichnet, daß man Cobaltsalze in Gegenwart von Butadien mit dem aktivierten Magnesium umsetzt.

15. Verfahren nach Anspruch 1 bis 5 zur Herstellung von Olefin-Komplex-Verbindungen der Übergangsmetalle, dadurch gekennzeichnet, daß man Übergangsmetallsalze in Gegenwart von Olefinen als Komplexbildner mit dem aktivierten Magnesium umsetzt.

16. Verfahren nach Anspruch 1 bis 5 und 12 zur Herstellung von Cycloocta-(1,5)dien-Komplexverbindungen des nullwertigen Nickels, Platins sowie Palladiums, dadurch gekennzeichnet, daß man Salze der betreffenden Übergangsmetalle in Gegenwart von Cycloocta-(1,5)dien als Komplexbildner mit dem aktivierten Magnesium umsetzt.

17. Verfahren nach Anspruch 1 bis 5 zur Herstellung von Tris(butadien) molybdän und Tris(butadien) wolfram, dadurch gekennzeichnet, daß man Salze der betreffenden Übergangsmetalle in Gegenwart von Butadien mit dem aktivierten Magnesium umsetzt.

18. Verfahren nach Anspruch 1 bis 4 und 6 zur Herstellung von Phosphan- bzw. Phosphitkomplexen der Übergangsmetalle MT des Periodensystems der Elemente der allgemeinen Formel $MTL_n$, wobei L für alkyl-, aryl-, sowie gemischte aryl-alkyl-substituierte Verbindungen des dreiwertigen Phosphors steht oder für Triorgano-Phosphite del allgemeinen Formel $P(OR)_3$, dadurch gekennzeichnet, daß man Salze der betreffenden Übergangsmetalle in Gegenwart von n Mol Phosphan oder Phosphit mit dem aktivierten Magnesium umsetzt.

19. Verfahren nach Anspruch 1 bis 4, 6 und 18 zur Herstellung von Tetrakistriphenylphosphan-Palladium, dadurch gekennzeichnet, daß man Palladiumsalze in Gegenwart von Triphenylphosphan mit dem aktivierten Magnesium umsetzt.

20. Verfahren nach Anspruch 1 bis 6 und 18 zur Herstellung von Ethylenbis(tri-o-tolylphosphit) nickel(0), dadurch gekennzeichnet, daß man Nickelsalze in Gegenwart von Ethylen und Tri-o-tolylphosphit mit dem aktivierten Magnesium umsetzt.

21. Verfahren nach Anspruch 1 bis 4, 6 und 18 zur Herstellung von Tris(tri-o-tolylphosphit) nickel(0), dadurch gekennzeichnet, daß man Nickelsalze in Gegenwart von Tri-o-tolyl-phosphit mit dem aktivierten Magnesium umsetzt.

22. Verfahren nach Anspruch 1 bis 5 zur Herstellung von gegebenenfalls substituierten Cyclopentadienyl-Übergangsmetall-Olefin-Komplexen, dadurch gekennzeichnet, daß man gegebenenfalls substituierte Cyclopentadienyl-Übergangsmetall-Komplexe, die noch ein oder mehrere salzartige Reste zusätzlich am Übergangsmetall gebunden enthalten, in Gegenwart der betreffenden Olefine mit dem aktivierten Magnesium umsetzt.

23. Verfahren nach Anspruch 1 bis 5 zur Herstellung von ($\mu$-dichloro) (Biscyclopentadienyl-Titan) fulven, dadurch gekennzeichnet, daß man Titantetrachlorid · 2 THF in Gegenwart von Cyclopentadien mit dem aktivierten Magnesium umsetzt.

24. Verfahren nach Anspruch 1 bis 5 zur Herstellung von gegebenenfalls substituierten Bis-(Cyclopentadienyl) Titandichlorid-Verbindungen, dadurch gekennzeichnet, daß man Titantetrachlorid · 2 THF in Gegenwart von gegebenenfalls substituiertem Cyclopentadien mit den aktivierten Magnesium umsetzt.

25. $\eta^5$-Indenylcobalt-$\eta^5$ Cyclopentadienyl.
26. $\eta^5$-Methylcyclopentadienylcobalt-cycloocta-(1,5)-dien.
27. $\eta^5$-tert.-Butylcyclopentadienylcobalt-cycloocta-(1,5)-dien.
28. $\eta^5$-Trimethylsilylindenylcobaltcycloocta-(1,5)-dien.
29. $\eta^5$-Phenylcyclopentadienylcobaltcycloocta-(1,5)-dien.
30. $\eta^5$-Indenylcobaltnorbornadien.
31. Verfahren zur Herstellung von Komplexverbindungen der Übergangsmetalle, dadurch gekennzeichnet, daß man Übergangsmetallsalze in Gegenwart komplexbildender Liganden mit Magnesium umsetzt, dem eine katalytische Menge eines eine oder zwei Methylgruppen oder Phenylgruppen enthaltenden Derivats von Anthracen oder deren Addukt an Magnesium als Aktivator zugesetzt wurde.

**Claims**

1. A process for the production of complex compounds of the transition metals, characterized in that transition metal salts are reacted with magnesium, to which a catalytic quantity of anthracene and/or magnesium anthracene has been added as activator, in the presence of complexing ligands.

2. A process as claimed in Claim 1, characterized in that the magnesium is treated in an ultrasonic bath with a catalytic quantity of anthracene and/or magnesium anthracene.

3. A process as claimed in Claims 1 and 2, characterized in that the transition metals used are those of Groups IVB to VIIIB of the Periodic System of Elements.

4. A process as claimed in Claims 1 to 3, characterized in that electron donors which form penetration complexes with transition metals are used as the complexing ligands.

5. A process as claimed in Claims 1 to 4, characterized in that compounds containing C-C-multiple bonds, such as olefins, substituted olefins, cyclic olefins, conjugated olefin systems, polyolefins, alkines and cycloalkines, are used as the complexing ligands.

6. A process as claimed in Claims 1 to 4, characterized in that the complexing ligands used are those containing free electron pairs, such as phosphanes, phosphites, arsanes, triorganyl antimony compounds, pyridines, dipyridines and carbon monoxide.

7. A process as claimed in Claims 1 to 5 for the production of cyclopentadienyl compounds of the transition metals, characterized in that cyclopentadiene and/or cyclopentadiene derivatives is/are used as the complexing agent.

8. A process as claimed in Claims 1 to 5 and 7 for the production of optionally substituted cyclopentadienyl iron compounds (ferrocenes), characterized in that iron salts are reacted with the activated magnesium in the presence of cyclopentadiene and/or cyclopentadiene derivatives.

9. A process as claimed in Claims 1 to 5 and 7 for the production of cyclopentadienyl cobalt complexes (cobaltocenes), characterized in that cobalt salts are reacted with the activated magnesium in the presence of cyclopentadiene and/or cyclopentadiene derivatives, the molar ratio of complexing agent to cobalt amounting to $\leq 3$.

10. A process as claimed in Claims 1 to 5 for the production of cyclopentadienyl cobalt olefin half-sandwich compounds, characterized in that cyclopentadiene or derivatives thereof and another olefin are used as complexing agent, in the presence of which cobalt salts are reacted with the activated magnesium.

11. A process as claimed in Claims 1 to 5 and 7 for the production of cyclopentadienyl cobalt cyclopentadiene compounds, characterized in that cobalt salts are reacted with the activated magnesium in the presence of cyclopentadiene and/or cyclopentadiene derivatives, the molar ratio of complexing agent to cobalt amounting to $\geq 4$.

12. A process as claimed in Claims 1 to 5 and 7 for the production of optionally substituted cyclopentadienyl cobalt cycloocta-(1,5)-diene compounds, characterized in that cyclopentadiene or derivatives thereof and cycloocta-(1,5)-diene are jointly used as complexing agent on the cobalt.

13. A process as claimed in Claims 1 to 6 for the production of $\eta^3$-allyl complexes of nickel and cobalt, characterized in that salts of these transition metals are reacted with the activated magnesium in the presence of 1,3-dienes and, optionally, other complexing agents.

14. A process as claimed in Claims 1 to 5 for the production of 5-methyl-$\eta^3$-heptadienyl cobalt butadiene, characterized in that cobalt salts are reacted with the activated magnesium in the presence of butadiene.

15. A process as claimed in Claims 1 to 5 for the production of complex olefin compounds of the transition metals, characterized in that transition metal salts are reacted with the activated magnesium in the presence of olefins as complexing agents.

16. A process as claimed in Claims 1 to 5 and 12 for the production of complex cycloocta-(1,5)-diene compounds of nonvalent nickel, platinum and palladium, characterized in that salts of the transition metals in question are reacted with the activated magnesium in the presence of cycloocta-(1,5)-diene as complexing agent.

17. A process as claimed in Claims 1 to 5 for the production of tris-(butadiene) molybdenum and tris-(butadiene) tungsten, characterized in that salts of the transition metals in question are reacted with the activated magnesium in the presence of butadiene.

18. A process as claimed in Claims 1 to 4 and 6 for the production of phosphane or phosphite complexes of the transition metals MT of the Periodic System of Elements corresponding to the general formula $MTL_n$, where L represents alkyl-, aryl- and mixed aryl-alkyl-substituted compounds of trivalent phosphorus or triorganophosphites corresponding to the general formula $P(OR)_3$, characterized in that salts of the transition metals in question are reacted with the activated magnesium in the presence of n moles of phosphane or phosphite.

19. A process as claimed in Claims 1 to 4, 6 and 18 for the production of tetrakis-triphenylphosphane palladium, characterized in that palladium salts are reacted with the activated magnesium in the presence of triphenyl phosphane.

20. A process as claimed in Claims 1 to 6 and 18 for the production of ethylene-bis-(tri-o-tolylphosphite) nickel (0) characterized in that nickel salts are reacted with the activated magnesium in the presence of ethylene and tri-o-tolylphosphite.

21. A process as claimed in Claims 1 to 4, 6 and 18 for the production of tris-(tri-o-tolylphosphite) nickel (0), characterized in that nickel salts are reacted with the activated magnesium in the presence of tri-o-tolylphosphite.

22. A process as claimed in Claims 1 to 5 for the production of optionally substituted cyclopentadienyl transition metal olefin complexes, characterized in that optionally substituted cyclopentadienyl transition metal complexes, which contain one or more salt-like residues additionally bound to the transition metal are reacted with the activated magnesium in the presence of the particular olefins.

23. A process as claimed in Claims 1 to 5 for the production of (μ-dichloro) (bis-cyclopentadienyl-titanium)-fulvene, characterized in that titanium tetrachloride · 2 THF is reacted with the activated magnesium in the presence of cyclopentadiene.

24. A process as claimed in Claims 1 to 5 for the production of optionally substituted bis-(cyclopentadienyl) titanium dichloride compounds, characterized in that titanium tetrachloride · 2 THF is reacted with the activated magnesium in the presence of optionally substituted cyclopentadiene.

25. $\eta^5$-indenylcobalt-$\eta^5$-cyclopentadienyl.

26. $\eta^5$-methylcyclopentadienyl cobalt cycloocta-(1,5)-diene.

27. $\eta^5$-tert.-butylcyclopentadienyl cobalt cycloocta-(1,5)-diene.

28. $\eta^5$-trimethylsilyl indenyl cobalt cycloocta-(1,5)-diene.

29. $\eta^5$-phenyl cyclopentadienyl cobalt cycloocta-(1,5)-diene.

30. $\eta^5$-indenyl cobalt norbornadiene.

31. A process for the production of complex compounds of the transition metals, characterized in that transition metal salts are reacted with magnesium, to which a catalytic quantity of an anthracene derivative containing one or two methyl or phenyl groups or the adduct thereof with magnesium has been added as activator, in the presence of complexing ligands.

**Revendications**

1. Procédé de préparation de complexes de métaux de transition, caractérisé en ce qu'on fait réagir des sels de métaux de transition, en présence de ligands complexants, avec du magnésium qui est additionné d'une quantité catalytique d'anthracène et/ou d'anthracène magnésium en tant qu'activateur.

2. Procédé selon la revendication 1, caractérisé en ce qu'on traite le magnésium dans un bain à ultrasons avec une quantité catalytique d'anthracène et/ou d'anthracène magnésium.

3. Procédé selon les revendications 1 et 2, caractérisé en ce qu'on met en œuvre comme métaux de transition ceux des groupes IVB et VIIIB de la classification périodique des éléments.

4. Procédé selon les revendications 1 à 3, caractérisé en ce qu'on met en œuvre comme ligands complexants des composés donneurs d'électrons qui forment des complexes de pénétration avec les métaux de transition.

5. Procédé selon les revendications 1 à 4, caractérisé en ce qu'on met en œuvre comme ligands complexants des composés avec des liaisons C-C multiples tels que des oléfines, des oléfines substituées, des oléfines cycliques, des systèmes oléfiniques conjugués, des polyoléfines, des alcynes et des cycloalcynes.

6. Procédé selon les revendications 1 à 4, caractérisé en ce qu'on met en œuvre comme ligands complexants ceux comportant des paires d'électrons libres tels que les phosphanes, les phosphites, les arsanes, les composés de triorganylantimoine, les pyridines, les dipyridines et l'oxyde de carbone.

7. Procédé selon les revendications 1 à 5 pour la préparation de composés cyclopentadiényliques des métaux de transition, caractérisé en ce qu'on met en œuvre du cyclopentadiène et/ou des dérivés de cyclopentadiène comme complexants.

8. Procédé selon les revendications 1 à 5 et 7 pour la préparation de composés cyclopentadiényliques du fer (ferrocènes) éventuellement substitués, caractérisé en ce qu'on fait réagir des sels de fer avec le magnésium activé en présence de cyclopentadiène et/ou de dérivés de cyclopentadiène.

9. Procédé selon les revendications 1 à 5 et 7 pour la préparation de complexes de cyclopentadiényl-cobalt (cobaltocènes), caractérisé en ce qu'on fait réagir des sels de cobalt, en présence de cyclopentadiène et/ou de dérivés de cyclopentadiène, avec le magnésium activé, réaction pendant laquelle le rapport molaire de complexant à cobalt s'élève à $\leq 3$.

10. Procédé selon les revendications 1 à 5 pour la préparation de composés en semi-sandwich cyclopentadiényl-cobalt-oléfine, caractérisé en ce qu'on met en œuvre du dicyclopentadiène ou ses dérivés et une autre oléfine en tant que complexants en présence desquels on fait réagir des sels de cobalt avec le magnésium activé.

11. Procédé selon les revendications 1 à 5 et 7 pour la préparation de composés de cyclopentadiényl-cobalt-cyclopentadiène, caractérisé en ce qu'on fait réagir des sels de cobalt, en présence de cyclopentadiène et/ou de dérivés de cyclopentadiène, avec le magnésium activé, opération pendant laquelle le rapport molaire de complexant à cobalt s'élève à $\geqslant 4$.

12. Procédé selon les revendications 1 à 5 et 7 pour la préparation de composés de cyclopentadiényl-cobalt-cycloocta(1,5)diène éventuellement substitués, caractérisé en ce qu'on met simultanément en œuvre du cyclopentadiène ou ses dérivés et du cyclo(1,5)octadiène comme complexants sur le cobalt.

13. Procédé selon les revendications 1 à 6 pour la préparation de complexes $\eta^3$-allyliques du nickel et du cobalt, caractérisé en ce qu'on fait réagir des sels de ces métaux de transition avec le magnésium activé en présence de (1,3)-diènes et éventuellement d'autres complexants.

14. Procédé selon les revendications 1 à 5 pour la préparation de 5-méthyl-$\eta^3$-heptadiénylcobaltbutadiène, caractérisé en ce qu'on fait réagir des sels de cobalt avec le magnésium activé en présence de butadiène.

15. Procédé selon les revendications 1 à 5 pour la préparation de complexes oléfiniques des métaux de transition, caractérisé en ce qu'on fait réagir des sels de métaux de transition avec le magnésium activé en présence d'oléfines en tant que complexants.

16. Procédé selon les revendications 1 à 5 et 12 pour la préparation de complexes de cycloocta-(1,5)-diène du nickel, du platine ainsi que du palladium de valence zéro, caractérisé en ce qu'on fait réagir des sels des métaux de transition concernés avec le magnésium activé en présence de cycloocta-(1,5)-diène comme complexant.

17. Procédé selon les revendications 1 à 5 pour la préparation de tris(butadiène)molybdène et de tris(butadiène)tungstène, caractérisé en ce qu'on fait réagir des sels des métaux de transition concernés avec le magnésium activé en présence de butadiène.

18. Procédé selon les revendications 1 à 4 et 6 pour la préparation de complexes de phosphane, respectivement de phosphite des métaux de transition MT de la classification périodique des éléments répondant à la formule générale $MTL_n$, L représentant des composés du phosphore trivalent substitués par un alkyle, un aryle ainsi que par un aryl-alkyle mixte ou des triorganophosphites de formule générale $P(OR)_3$, caractérisé en ce qu'on fait réagir des sels des métaux de transition concernés avec le magnésium activé ave n moles de phosphane ou de phosphite.

19. Procédé selon les revendications 1 à 4, 6 et 18 pour la préparation de tétrakistriphénylphosphane palladium, caractérisé en ce qu'on fait réagir des sels de palladium avec le magnésium activé en présence de triphénylphosphane.

20. Procédé selon les revendications 1 à 5 et 18 pour la préparation d'éthylènebis(tri-o-tolylphosphite)nickel (0), caractérisé en ce qu'on fait réagir des sels de nickel avec le magnésium activé en présence d'éthylène et de tri-o-tolylphosphite.

21. Procédé selon les revendications 1 à 4, 6 et 18 pour la préparation de tris(tri-o-tolylphosphite)nickel (0), caractérisé en ce qu'on fait réagir des sels de nickel avec le magnésium activé en présence de tri-o-tolylphosphite.

22. Procédé selon les revendications 1 à 5 pour la préparation de complexes de cyclopentadiényl-métal de transition-oléfine éventuellement substitués, caractérisé en ce qu'on fait réagir des complexes cyclopentadiényliques de métaux de transition éventuellement substitués qui contiennent encore un ou plusieurs restes salins liés en plus au métal de transition, avec le magnésium activé en présence des oléfines concernées.

23. Procédé selon les revendications 1 à 5 pour la préparation de ($\mu$-dichloro) (biscyclopentadiényltitane) fulvène, caractérisé en ce qu'on fait réagir du tétrachlorure de titane · 2 THF avec le magnésium activé en présence de cyclopentadiène.

24. Procédé selon les revendications 1 à 5 pour la préparation de composés de bis-(cyclopentadiényl)dichlorure de titane éventuellement substitués, caractérisé en ce qu'on fait réagir du tétrachlorure de titane · 2 THF avec le magnésium activé en présence de cyclopentadiène éventuellement substitué.

25. $\eta^5$-indénylcobalt-$\eta^5$ cyclopentadiényle.

26. $\eta^5$-méthylcyclopentadiénylcobalt-cycloocta-(1,5)-diène.

27. $\eta^5$-tert.-butylcyclopentadiénylcobalt-cycloocta-(1,5)-diène.

28. $\eta^5$-triméthylsilylindénylcobaltcycloocta-(1,5)-diène.

29. $\eta^5$-phénylcyclopentadiénylcobaltcycloocta-(1,5)-diène.

30. $\eta^5$-indénylcobaltnorbornadiène.

31. Procédé de préparation de complexes des métaux de transition, caractérisé en ce qu'on fait réagir des sels de métaux de transition, en présence de ligands complexants, avec du magnésium qui a été additionné d'une quantité catalytique d'un dérivé de l'anthracène contenant un ou deux groupes méthyles ou des groupes phényles ou de son produit d'addition sur le magnésium en tant qu'activateur.